# EUROPEAN PATENT SPECIFICATION

(11) **EP 3 712 247 B1**
(45) Date of publication and mention of the grant of the patent: **05.03.2025**
(21) Application number: 18878958.0
(22) Date of filing: 14.11.2018
(51) Int. Cl.: C12N 1/00, C02F 3/00, C02F 3/34, C12N 1/20, C02F 101/30

(54) **METHOD FOR TREATING OIL- AND FAT-CONTAINING WASTEWATER**
VERFAHREN ZUR BEHANDLUNG VON ÖL- UND FETTHALTIGER ABWÄSSER
PROCÉDÉ DE TRAITEMENT D'EAUX USÉES CONTENANT DES HUILES ET DES MATIÈRES GRASSES

(30) Priority: 14.11.2017 JP 2017218942; 26.10.2018 JP 2018202323
(43) Date of publication of application: 23.09.2020
(73) Proprietor: National University Corporation Tokai National Higher Education and Research System, Nagoya-shi Aichi 464-8601 (JP)
(72) Inventor: HORI, Katsutoshi, Nagoya-shi Aichi 464-8601 (JP)
(74) Representative: Uexküll & Stolberg
(86) International application number: PCT/JP2018/042197
(87) International publication number: WO 2019/098255

(56) References cited:
- WO-A1-2016/005771
- JP-A- 2002 113 489
- JP-A- 2002 126 785
- JP-A- 2003 251 396
- JP-A- 2011 160 713
- JP-A- 2011 182 782
- JP-A- 2017 177 031
- JP-A- H08 197 086
- US-A1- 2003 203 470
- US-A1- 2015 024 470

## Description

### [Technical Field]

The present invention relates to an oil- and fat-containing wastewater treatment method using a microbial formulation with an ability to decompose oil and fat, and a related system and apparatus are further disclosed but not claimed.

### [Background Art]

Decomposition of oil and fat by microorganisms is utilized in wastewater treatment and the like. A large quantity of oil content contained in wastewater from food plants and oil and fat refining factories has the potential to significantly reduce the treatment efficiency of various wastewater treatment processes, such as activated sludge process, membrane bioreactor (MBR), and anaerobic digestion. In this regard, many factories that discharge such wastewater have a dissolved air flotation apparatus or the like installed for pretreatment in these treatment processes.

While a dissolved air flotation apparatus removes oil content by separating solids from liquids, such an apparatus faces the following problems: (1) a large quantity of retrieved oily sludge needs to be treated as industrial waste; (2) to make oil content float up, a large quantity of flocculant needs to be used and buoyancy needs to be imparted by providing air bubbles through aeration; (3) factories that are a source of odor generation and are located in the vicinity of residential areas must install a deodorizing apparatus; (4) due to being readily affected by variations in the amount of wastewater and oil content, the operation and management of the apparatus are labor intensive; and (5) as a result thereof, the cost associated with industrial waste treatment, operation, management, maintenance, and the like is significant.

In this regard, there is a pressing need to establish a technology that would eliminate oil content in wastewater without separation. Under such a circumstance, application of oil and fat decomposition technologies using microorganisms has been attempted. JP 2017 177031 A discloses a method for treating wastewater containing oil and fat by adding a microbial formulation to the water in a vessel.

In this regard, the inventor has reported a novel microorganism Burkholderia arboris SL1B1 strain that secretes an oil and fat hydrolase, i.e., lipase, as a microorganism which efficiently treats oil- and fat-containing wastewater (Patent Literature 1). Furthermore, the inventor has reported that oil and fat is efficiently decomposed by combined use of said Burkholderia arboris with a microorganism (e.g., Candida cylindracea) that decomposes a product of hydrolysis by lipase, i.e., glycerol (Patent Literature 2, Non Patent Literature 1). The inventor has also reported that oil and fat can also be efficiently decomposed by combined use of said Burkholderia arboris with Yarrowia lipolytica, which does not produce lipase but decomposes free fatty acids (Patent Literature 3). Such an ability to decompose oil and fat can replace a dissolved air flotation apparatus.

However, no matter how high the ability of the used microbial formulation to decompose is, if used incorrectly, the intended effect cannot be attained. In fact, microorganisms found to have the ability to decompose in a laboratory frequently have no effect when used in practice. The primary reason thereof is in the significant difference between the laboratory conditions and actual practice conditions. In a laboratory, only the microorganism of interest is seeded and cultured in a sterilized and closed system in many cases, and microorganisms are often cultured in batches. The results would be completely different between batch and continuous reactions. In this regard, continuous reactions are also performed in a laboratory in some cases. However, in many practical settings, inflow and outflow of wastewater to and from an oil and fat decomposition vessel are continuous, whereas a microbial formulation is not continuously added, but intermittently added at regular intervals. In such a case, the operational theory of conventional continuous reactors would not hold.

Since wastewater is treated in an open system, the treatment is performed in an environment with an infinite number of assorted microorganisms besides the added microorganism. A methodology for stably establishing (a state where the microorganism of interest is effectively present without being selected out in a target environment) a microorganism of interest such as an oil and fat decomposing microorganism in such a microorganism community does not exist. Moreover, wastewater continuously flows out with a constant retention time, so that the added microorganism of interest would be washed out if the microorganism cannot win the competition for survival against infinite number of other microorganisms and proliferate at a faster specific proliferation rate than the retention time. For this reason, the expected decomposition effect is often not attained, which is an object to be addressed in the field of environmental biotechnology.

A method of avoiding washout includes a method of immobilizing a microorganism to a carrier, but such a method results in new problems such as the issue of oil adhering to the carrier in wastewater with a large quantity of oil, as well as generation of new waste, i.e., carrier, and cost for the carrier.

Furthermore, attention was hardly given to how a microbial formulation is used up to this point. In most cases, the amount and interval of addition were adjusted by feel while observing the effect. In fact, it was actually challenging to establish a defined methodology, due to the diversity in the quantity and oil and fat concentration of wastewater depending on the setting. In addition, conventional technologies faced problems such as difficulty in applying the same microbial formulation to a wide range of oil and fat concentrations. In particular, it is challenging to apply a microbial formulation to a high concentration of oil and fat discharged from a food plant, oil and fat refining factory, or the like. Even if it was possible, a high microbial concentration of 10⁹ cells/mL or greater is required.

### [Citation List]

### [Patent Literature]

[PTL 1] Japanese Laid-Open Publication No. 2010-227858
[PTL 2] Japanese Laid-Open Publication No. 2010-227849
[PTL 3] Japanese Laid-Open Publication No. 2013-146689

### [Non Patent Literature]

[NPL 1] Journal of Bioscience and Biotechnology, Vol. 107, No.4, 401-408, 2009

### [Summary of Invention]

### [Solution to Problem]

The present invention provides an oil- and fat-containing wastewater treatment method for effectively reducing the concentration of oil and fat in wastewater that continuously flows in and out and contains various microorganisms by using a microbial formulation with the ability to decompose oil and fat, and a related system and apparatus are disclosed but not claimed.

As a result of a series of diligent studies to achieve the aforementioned object, the inventor found that when a microbial formulation is added to an oil and fat decomposition vessel where oil- and fat-containing wastewater continuously flows in and out, the added microorganism does not necessarily need to proliferate in the oil and fat decomposition vessel, and oil and fat can be decomposed if there is a certain number of the microorganisms, which is fundamentally different from conventional continuous culture.

The present invention was completed after further studies based on these findings and provides an oil- and fat-containing wastewater treatment method according to claim 1.

### [Advantageous Effects of Invention]

The method of the invention can effectively reduce the concentration of oil and fat in wastewater that continuously flows in and out and contains various microorganisms by continuously or intermittently adding a microbial formulation with the ability to decompose oil and fat. The present invention is characterized in that a microorganism does not necessarily need to proliferate in an oil and fat decomposition vessel, and oil and fat can be decomposed even if a microorganism cannot proliferate.

The present invention is a process where various microorganisms other than the added microorganism are present, and oil- and fat-containing wastewater continuously flows in and out, so that the added microorganism tends not to proliferate, but the microorganism can be established in an oil and fat decomposition vessel.

The method of the invention is compatible with a wide range of oil and fat concentrations, and is cost effective because a microbial formulation with a lower concentration than conventional art can be used.

In addition, or alternatively, the method of the invention can attain an environmentally friendly effect because the amount of added microorganism in water flowing out from a decomposition vessel is less than conventional art, while sufficiently decomposing oil and fat in wastewater.

### [Brief Description of Drawings]

[Figure 1] Figure **1** is a graph showing the effect of dissolved oxygen concentration (DO) on oil and fat decomposition by microorganisms.
[Figure 2] Figure **2** is a graph showing the effect of pH on oil and fat decomposition by microorganisms.
[Figure 3] Figure **3** is a graph showing the effect of temperature on oil and fat decomposition by microorganisms.
[Figure 4] Figure **4** is a graph showing the effect of C/N on oil and fat decomposition by microorganisms.
[Figure 5] Figure **5** is a graph showing the effect of N/P on oil and fat decomposition by microorganisms.
[Figure 6] Figure **6** is a graph showing the effect of the concentration of added microorganisms upon intermittent addition in low concentration oil and fat (normal hexane value of about 300 mg/L) wastewater.
[Figure 7] Figure **7** is a graph showing the effect of the concentration of added microorganisms upon intermittent addition in moderate concentration oil and fat (normal hexane value of about 3000 mg/L) wastewater.
[Figure 8] Figure **8** is a graph showing the effect of the concentration of added microorganisms upon intermittent addition in high concentration oil and fat (normal hexane value of about 10000 mg/L) wastewater.
[Figure 9] Figure **9** is a graph showing the effect of the concentration of added microorganisms upon intermittent addition in ultrahigh concentration oil and fat (normal hexane value of about 30000 mg/L) wastewater.
[Figure 10] Figure **10** is a graph showing the effect of the concentration of added microorganisms upon continuous addition in low concentration oil and fat (normal hexane value of about 300 mg/L) wastewater.
[Figure 11] Figure **11** is a graph showing the effect of the concentration of added microorganisms upon continuous addition in moderate concentration oil and fat (normal hexane value of about 3000 mg/L) wastewater.
[Figure 12] Figure **12** is a graph showing the effect of the concentration of added microorganisms upon continuous addition in high concentration oil and fat (normal hexane value of about 10000 mg/L) wastewater.
[Figure 13] Figure **13** is a graph showing the effect of the concentration of added microorganisms upon continuous addition in ultrahigh concentration oil and fat (normal hexane value of about 30000 mg/L) wastewater.
[Figure 14] Figure **14** is a graph showing the effect of retention time in low concentration oil and fat (normal hexane value of about 300 mg/L) wastewater.
[Figure 15] Figure **15** is a graph showing the effect of retention time in moderate concentration oil and fat (normal hexane value of about 3000 mg/L) wastewater.
[Figure 16] Figure **16** is a graph showing the effect of retention time in high concentration oil and fat (normal hexane value of about 10000 mg/L) wastewater.
[Figure 17] Figure **17** is a graph showing the effect of retention time in ultrahigh concentration oil and fat (normal hexane value of about 30000 mg/L) wastewater.
[Figure 18] Figure **18** is a diagram showing a system for the treatment method of the invention.

### [Description of Embodiments]

The present invention is described in detail hereinafter. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

The definitions of the terms and/or basic technical concepts that are particularly used herein are described hereinafter when appropriate.

As used herein, "comprise" encompasses the meaning of "essentially consisting of" as well as the meaning of "consist of".

As used herein, normal hexane (n-Hex) value is the amount of nonvolatile substance extracted with normal hexane. This is an indicator that indicates the amount of oil content (oil and fat, hydrolysis product thereof, etc.) in water. An n-Hex value can be found, for example, in accordance with JIS K 0102. Alternatively, the n-Hex value of the invention can be, for example, an equivalent value that is measured using a thermo-responsive polymer (e.g., poly(N-isopropylacrylamide) (PNIPPAm)). PNIPPAm extracted substances can be measured using a known kit, such as an oil content measuring reagent set that is available from Kyoritsu Chemical-Check Lab., Corp (Tokyo, Japan).

The oil- and fat-containing wastewater treatment method of the invention is characterized by comprising decomposing oil and fat by continuously or intermittently adding a microbial formulation with an ability to decompose oil and fat to an oil and fat decomposition vessel where oil- and fat-containing wastewater continuously flows in and out, wherein an amount of the microbial formulation added is an amount at which a microorganism can be established in the oil and fat decomposition vessel, and a microorganism other than the microorganism comprised in the microbial formulation is present in oil- and fat-containing wastewater.

The present invention is a technology for reducing an n-Hex value derived from oil and fat in wastewater and hydrolysis product thereof under a suitable process condition.

The oil- and fat-containing wastewater in the present invention is not particularly limited, as long as it is wastewater containing oil and fat. Examples thereof include wastewater from a restaurant, hospital, hotel, or the like, domestic wastewater, industrial wastewater discharged from a food processing plant, oil and fat processing plant, and the like.

The oil and fat in wastewater in the present invention is not particularly limited. Examples thereof include vegetable oil and fat (cottonseed oil, rapeseed oil, soybean oil, corn oil, olive oil, safflower oil, rice oil, sesame oil, palm oil, coconut oil, peanut oil, and the like), animal oil and fat (lard, beef tallow, milk fat, and the like), fish oil, processed products of such oil and fat (margarine, shortening, butter, and the like), and the like.

The n-Hex value of oil- and fat-containing wastewater in the present invention is preferably 300 to 30000 mg/L. The present invention is for treating oil- and fat-containing wastewater with a mean n-Hex value exceeding 300 mg/L. In the method of the invention the n-Hex value of oil- and fat-containing wastewater to be treated is measured to determine the amount of microbial formulation to be added based on the measured value. An n-Hex value may be identified in the present invention in accordance with JIS K 0102, or by using an equivalent value that is measured using poly(N-isopropylacrylamide) (PNIPPAm).

A microorganism other than the microorganism comprised in a microbial formulation is present in the oil- and fat-containing wastewater in the present invention. Since the method of the invention is generally performed in an open system, the method is performed in an environment with various microorganisms in oil- and fat-containing wastewater. Examples of the number of microorganisms in oil- and fat-containing wastewater include 1 × 10⁶ cells/mL or greater, 1 × 10⁷ cells/mL or greater, 1 × 10⁸ cells/mL or greater, 1 × 10⁹ cells/mL or greater, and the like. Such an environment with various microorganisms therein is an environment where it is difficult for a microorganism in the added microbial formulation to proliferate, unlike a pure culture.

In the present invention, oil- and fat-containing wastewater continuously flows in and out, to and from an oil and fat decomposition vessel. "Continuous" in this context does not only mean that oil- and fat-containing wastewater is ceaselessly flowing in and flowing out, but also encompasses cases where inflow and outflow momentarily stop. It should be noted that "continuous" in the present invention does not encompass a system where a microorganism is added to, proliferates, and performs decomposition and treatment in a closed tank, as batch treatment.

A microbial formulation can be used without any particular limitation, as long as the formulation has an ability to decompose oil and fat. As a microorganism in a microbial formulation, one species alone can be used, or two or more species can be mixed and used. If two or more species are mixed and used, it is desirable to use a combination of microorganisms that can coexist. A microbial formulation can also comprise a symbiotic bacteria that facilitates decomposition of oil and fat.

Examples of microbial formulations include a microbial formulation with an ability to decompose 80% or more of oil and fat and fatty acid of a hydrolysis product corresponding to an N-Hex value = 10000 mg/L within 72 hours, preferably within 48 hours, more preferably within 36 hours, and still more preferably within 24 hours, under conditions of 22 to 35°C, pH = 5.5 to 8.5, DO ≥ 0.1 mg/L, and batch culture.

A microorganism that produces lipase, and a microorganism that decomposes fatty acid and/or glycerol (which is product from decomposition of oil and fat by lipase) can be suitably used in a microbial formulation. As such a microorganism, not only a microorganism having such a property independently, but also a microorganism with a plurality of such properties (e.g., microorganism that produces lipase and decomposes fatty acid) can be used.

A microorganism that produces lipase is, for example, eubacteria, yeast, filamentous fungus, or the like, preferably eubacteria or yeast, and more preferably eubacteria. Examples of eubacteria that can be used include Bacillus bacteria, Corynebacterium bacteria, Rhodococcus bacteria, Burkholderia bacteria, Acinetobacter bacteria, Pseudomonas bacteria, Alcaligenes bacteria, Rhodobacter bacteria, Ralstonia bacteria, Acidovorax bacteria, Serratia bacteria, Flavobacterium bacteria, and the like. In particular, Burkholderia bacteria, especially Burkholderia arboris (e.g., SL1B1 strain (NITE P-724) and the like) is preferred.

A microorganism that decomposes (assimilates) glycerol is, for example, eubacteria, yeast, filamentous fungus, or the like, preferably eubacteria or yeast, and more preferably yeast. As yeast, Candida yeast, especially Candida cylindracea (e.g., SL1B2 strain (NITE P-714) and the like) is preferred.

A microorganism that decomposes (assimilates) fatty acid is, for example, eubacteria, yeast, filamentous fungus, or the like, preferably eubacteria or yeast, and more preferably yeast. As yeast, Yarrowia yeast, Cryptococcus yeast, Trichosporon yeast, and Hansenula yeast, especially Yarrowia lipolytica (e.g., 1A1 strain (NITE BP-1167) and the like) are preferred.

Specific examples of microorganisms used for manufacturing a microbial formulation include Burkholderia arboris, Candida cylindracea, and Yarrowia lipolytica. It is desirable to use two or three species thereof in combination.

A microorganism can be manufactured by culturing with a known method. Examples of forms of a microbial formulation include a liquid state, dry state, paste-like state, and state of being adsorbed to powder/granules such as sawdust. A microbial formulation can also be used while being immobilized to a carrier.

Washout can be avoided by using such a carrier. Any material of a carrier can be used without any particular limitation, as long as a microorganism can be immobilized. Examples thereof include carbon fiber (PAN-based, pitch-based, phenol resin-based, or the like), polyethylene resin, polypropylene resin, polyurethane resin, polystyrene resins, polyvinyl chloride resin, polyvinyl acetate resin, polyvinyl alcohol resin, polyethylene glycol resin, acrylic resin, gelatin, sodium alginate, carrageenan, dextrin, ceramic, silicon, metal, composites thereof, and the like. To increase the immobilization rate and working efficiency of microorganisms, it is preferable to use a porous or fibrous carrier. A microorganism may also be covered with a gel-like carrier. Examples of shapes of a carrier include a cube shape, rectangular cuboid shape, cylindrical shape, spherical shape, disc-like shape, sheet-like shape, membranous shape, and the like. However, the method of the invention can attain a sufficient oil and fat decomposition effect without using such a carrier, so that the use of a carrier is not essential.

A method of adding a microbial formulation to an oil and fat decomposition vessel employs either a continuous or intermittent system. An intermittent system refers to a system that periodically adds a microbial formulation.

The amount of microbial formulation added to an oil and fat decomposition vessel is an amount at which a microorganism can be (stably) established in the oil and fat decomposition vessel, wherein "establish" refers to a state where a microorganism of interest is present in an oil and fat decomposition vessel, without being selected out, to the extent oil and fat can be decomposed. Specifically, this refers to a state in which an added microorganism is not washed out even under a condition where wastewater continuously flows out.

A carrier is not used in the oil and fat decomposition method of the invention. The present invention unexpectedly enabled sufficient decomposition of oil and fat in wastewater while avoiding washout by determining the amount of microorganism to be added depending on the amount of oil and fat contained in wastewater to be treated and using a concentration of microorganisms in an oil and fat decomposition vessel that is lower than conventional art, without using a carrier. Washout refers to a phenomenon in which the bacterial proliferation rate cannot catch up to the outflow rate of a continuous system such that the concentration cannot be maintained. In general, an ideal continuous system is in a steady state, where the following equation holds. Amount of added microorganism (cell/h) + amount of proliferation of microorganisms (cell/h) = amount of microorganism outflow (cell/h), wherein amount of added microorganisms (cell/h) = microorganism concentration in microbial formulation (cell/L) × rate of adding microbial formulation (L/h), amount of proliferation of microorganisms (cell/h) = specific proliferation rate (1/h) × microorganism concentration in vessel (cell/L) × vessel capacity (L), and amount of microorganism outflow (cell/h) = microorganism concentration in water flowing out (cell/L) × wastewater outflow rate (L/h).

It was the general understanding in the art that the amount of added microorganisms is normally significantly lower than the amount of proliferation of microorganisms in the vessel, such that wastewater treatment is attempted by proliferated microorganisms. However, the inventor investigated whether microorganisms can be supplemented even if the added microorganisms do not proliferate, without contemplating proliferating microorganisms, and conceived of supplementing at the following amount: Amount of added microorganism = amount of microorganism outflow. If the added microorganisms do not proliferate, the following equation holds: microorganism concentration in microbial formulation × amount of microbial formulation added = microorganism concentration in water flowing out × oil and fat decomposition vessel volume, i.e., microorganism concentration in microbial formulation × amount of microbial formulation added/oil and fat decomposition vessel volume = concentration of added microorganism upon addition = microorganism concentration in water flowing out.

Specifically, the concentration of added microorganism would be responsible for oil and fat decomposition in wastewater. It was the understanding in the art that it is necessary to allow microorganisms to proliferate and achieve decomposition of oil and fat with the proliferated microorganisms in order to sufficient decompose oil and fat, but the inventor unexpectedly found that decomposition of oil and fat can be achieved with the concentration of added microorganisms without depending on proliferation of microorganisms. Of course, the advantage of the invention is similarly beneficial even if the added microorganisms proliferate.

Microorganisms generally consume a carbon source and convert an amount equal to the amount of consumption multiplied by the bacterial cell yield into a bacterial cell component to proliferate. Since the carbon source in decomposition of oil and fat is oil and fat, in theory, the bacterial cells proliferate with decomposition of oil and fat. The amount of proliferation is determined by bacterial cell yield. In many cases, this is about 0.5, but varies depending on the environmental conditions. Specifically, in terms of carbon balance, half of the decomposed oil and fat generally becomes proliferated bacterial cells, and the remaining half is utilized for the maintenance of bacterial cells as an energy source and mineralized to carbon dioxide. No proliferation basically means that the apparent bacterial cell yield is zero, indicating that all decomposed oil and fat is used for maintaining the bacterial cells, or proliferated bacterial cells die at the same rate or are selected out. This is generally an inconceivable phenomenon and not a common operation taught by biochemical engineering that is the theoretical basis of wastewater treatment. However, the inventor found that if competition for survival with other microorganisms is intense, such an operation is possible by balancing the amount of oil and fat with the amount of added microorganisms. If this balance is disrupted such that the amount of added microorganisms is too low relative the amount of oil and fat, the microorganisms would have room to proliferate, but excess oil and fat would remain. In contrast, if the amount of added microorganisms is too high relative to the amount of oil and fat, a carbon source required for the maintenance of bacterial cells cannot be secured, such that the microorganism concentration would decrease. Such a condition results in a state where added microorganisms are meaninglessly expended. Specifically, one of the most important aspects of the invention is the discovery of an amount of added bacterial cells that corresponds to the amount of oil and fat in wastewater, in which various microorganisms are present and the competition for survival is intense.

When a microbial formulation is intermittently added to an oil and fat decomposition vessel, the amount of microbial formulation added is an amount that results in preferably 5.0 × 10⁴ to 1.0 × 10⁷ cells/mL, more preferably 1.0 × 10⁵ to 1.0 × 10⁷ cells/mL, still more preferably 5.0 × 10⁵ to 5.0 × 10⁶ cells/mL, and most preferably 1.0 × 10⁶ to 3.0 × 10⁶ cells/mL with respect to wastewater in an oil and fat decomposition vessel. It is desirable to set the interval for adding periodically added microorganism between the retention time of wastewater in an oil and fat decomposition vessel and 24 hours. The amount of microbial formulation added in this regard can also be the concentration of the type of microorganism that is comprised the most in the microbial formulation.

When a microbial formulation is continuously added, FX, F, and IX are entered into the following equation to find SX.

Microbial formulation supply rate (FX): treated wastewater flow volume (F) = microorganism concentration after dilution from inflow into oil and fat decomposition vessel (IX): supplied microorganism concentration (SX).
IX is calculated to be preferably 5.0 × 10⁴ to 1.0 × 10⁷ cells/mL, more preferably 1.0 × 10⁵ to 1.0 × 10⁷ cells/mL, still more preferably 5.0 × 10⁵ to 5.0 × 10⁶ cells/mL, and most preferably 1.0 × 10⁶ to 3.0 × 10⁶ cells/mL with respect to wastewater in an oil and fat decomposition vessel. FX:F is typically, but is not limited to, 1:1000. The supplied microorganism concentration in this regard can also be the concentration of the type of microorganism that is comprised the most in the microbial formulation.

If a plurality of species of microorganisms are used, each microorganism can be added to an oil and fat decomposition vessel simultaneously or independently.

It is necessary to maintain the dissolved oxygen concentration (DO) of oil- and fat-containing wastewater in the oil and fat decomposition vessel according to the invention at 0.05 mg/L or greater, preferably 0.1 mg/L or greater, more preferably 0.5 mg/L or greater, and most preferably 1 mg/L or greater by aeration and agitation or the like.

The pH of oil- and fat-containing wastewater in the oil and fat decomposition vessel is according to the invention within the range of 4.5 to 9.0, preferably 5.5 to 8.5, and more preferably 6.0 to 8.0. If the pH is not within these ranges, pH may be appropriately adjusted by adding an acid or alkali.

It is necessary to maintain the temperature of oil- and fat-containing wastewater in the oil and fat decomposition vessel according to the invention within the range of 12°C to 42°C, preferably 15°C to 40°C, more preferably 15 to 37°C, still more preferably 18 to 37°C, and most preferably 20 to 35°C. If the temperature is not within these temperature ranges, the temperature may be appropriately adjusted by heating or cooling.

The present invention targets oil and fat decomposition where an HRT (hydraulic retention time) in the oil and fat decomposition vessel is 1 hour or greater. Specifically, the HRT in an oil and fat decomposition vessel is generally 12 hours or greater, preferably 18 hours or greater, more preferably 20 hours or greater, and still more preferably 24 hours or greater. If a reduction of 80% or greater in the n-Hex value is expected for wastewater with n-Hex exceeding 10000 mg/L, the HRT is generally 18 hours or greater, preferably 20 hours or greater, and more preferably 24 hours or greater. If a reduction of 80% or greater in the n-Hex value is expected for wastewater with an n-Hex value of 3000 mg/L or less, the HRT is generally 8 hours or greater, preferably 12 hours or greater, and more preferably 18 hours or greater. Typically, the HRT may be less than or equal to 48 hours in either treatment.

It is desirable that nitrogen is in wastewater flowing into an oil and fat decomposition vessel in a form that is available to microorganisms, preferably in a form including ammonium salt, nitrate, sulfate, or organic nitrogen compound, more preferably ammonium sulfate, urea, amino acid, or peptone, tryptone, casamino acid, or other peptides. The required amount of nitrogen is preferably estimated in advance from a lab scale or pilot scale test. The amount is generally in the range of C/N = 2 to 50 (wherein C refers to carbon only from n-Hex) and preferably C/N = 2 to 30, and more preferably C/N = 2 to 20. However, C/N is the weight ratio of n-Hex derived carbon atom to nitrogen atom comprised in wastewater. If the ratio is not within the above ranges due to insufficient nitrogen in wastewater, the required amount of nitrogen may be added in a form described above, preferably in a form of ammonium sulfate or urea. In another embodiment, nitrogen may be added by supplying as ammonium or an aqueous solution thereof concurrently with the automatic adjustment of pH in order to simultaneously control the decrease in pH accompanied by decomposition of oil and fat. In this regard, nitrogen may be supplied automatically or manually.

It is desirable that phosphorous (P) is present in wastewater flowing into an oil and fat decomposition vessel in a form that is available to microorganisms, preferable in a form of phosphate or nucleic acid, and more preferably phosphate. It is desirable that the required amount of phosphorous is estimated from a lab scale or pilot scale test. The amount is generally an amount resulting in N/P = 1 to 20 with respect to nitrogen in wastewater. However, N/P is the weight ratio of nitrogen atom to phosphorous atom comprised in wastewater. If the ratio is not within the above range due to insufficient phosphorous in wastewater, the required amount of phosphorous may be added in a form of phosphate. In this regard, phosphorous may be added automatically or manually.

The microorganism concentration in an oil and fat decomposition vessel is dependent on the oil and fat concentration in wastewater. The bacterial cell concentration is maintained higher for higher oil and fat concentration. Since a low concentration of added microorganisms results in active proliferation due to oil and fat in wastewater, the microorganism concentration may rather become high in some cases. In contrast, when the amount of added microorganisms is sufficient, the oil and fat concentration decreases sufficiently, such that the amount of microorganisms is maintained at a low concentration in some cases.

In case of foaming in the oil and fat decomposition vessel, defoaming measures such as reducing the HRT, showering, or adding a defoamer may be taken. However, some defoamers inhibit the growth of microorganisms, thus it is desirable to set the amount added while taking this into consideration.

When wastewater comprises chlorine, antibiotics, or the like to the extent that the growth of microorganisms is suppressed, a microbial formulation may be added after treatment to remove them, or at a later stage (e.g., after the equalization tank) at which the concentration of these growth suppressing substances is reduced while taking into consideration the entire wastewater treatment system.

For low concentration wastewater with an n-Hex value of water flowing in of about 300 mg/L or less, the n-Hex value of water flowing out from an oil and fat decomposition vessel is preferably 60 mg/L or less, and more preferably 30 mg/L or less. For moderate concentration wastewater with an n-Hex value of water flowing in of about 3000 mg/L, the n-Hex value of water flowing out from an oil and fat decomposition vessel is preferably 600 mg/L or less, more preferably 300 mg/L or less, still more preferably 150 mg/L or less, and most preferably 30 mg/L or less. For high concentration wastewater with an n-Hex value of water flowing in of about 10000 mg/L, the n-Hex value of water flowing out from an oil and fat decomposition vessel is preferably 1000 mg/L or less, more preferably 500 mg/L or less, still more preferably 100 mg/L or less, and most preferably 30 mg/L or less. For high concentration wastewater with an n-Hex value of water flowing in of about 30000 mg/L or greater, the n-Hex value of water flowing out from an oil and fat decomposition vessel is preferably 3000 mg/L or less, more preferably 1000 mg/L or less, and still more preferably 300 mg/L or less.

Further, the n-Hex value of oil- and fat-containing wastewater can be reduced by preferably 80% or greater, more preferably 90% or greater, still more preferably 95% or greater, and most preferably 99% or greater by the method of the invention.

As a result, the n-Hex value of water flowing out from an oil and fat decomposition vessel can be reduced in most wastewater to less than 30 mg/L, which is the reference value of release into the sewage system at many municipalities. If such a reference value is achieved, even the main treatment such as active sludge treatment in the later stage would not be needed in terms of just the n-Hex value.

While the conventional understanding of the art was that microorganisms needed to be sufficiently proliferated for oil and fat decomposition treatment using microorganisms in a continuous system, the inventor unexpectedly discovered that decomposition of oil and fat can be achieved without proliferation of microorganisms to the extent of conventional understanding. While it was conventionally understood that microorganisms needed to be proliferated to an amount that is several 100-fold or greater and several 1000-fold or less with respect to the amount of added microorganisms, the inventor found that wastewater can be successfully treated by proliferation of microorganisms to an amount that is only 100-fold or less or about several 10-fold with respect to the amount of added microorganisms. For example, the amount of added microorganisms in water flowing out is preferably 0.01-fold or greater and 100-fold or less, 0.01-fold or greater and 10-fold or less, 0.1-fold or greater and 100-fold or less, 0.1-fold or greater and 10-fold or less, 0.5-fold or greater and 100-fold or less, 0.5-fold or greater and 10-fold or less, 1-fold or greater and 100-fold or less, 1-fold or greater and 10-fold or less, and preferably 0.1-fold or greater and 10-fold or less with respect to the added amount. For this reason, in one embodiment, the amount of added microorganism in water flowing out from an oil and fat decomposition vessel can be 1 × 10⁸ cells/mL or less. This allowed the amount of microorganisms in water flowing out to be suppressed and the impact on the environment to be reduced. Since the amount of proliferation of microorganisms is dependent on the amount of oil in fat which is the feed (carbon source), the amount of microorganisms can be controlled by adding an amount of microorganisms in accordance with the amount of oil and fat in wastewater to achieve efficient decomposition of oil and fat. Information regarding the amount of microorganisms required for the amount of oil and fat to be treated did not exist in the past. According to the invention, the amount of microbial formulation added to the oil and fat decomposition vessel is 3.0 × 10⁴ to 1.0 × 10⁷ cells per 1 mg of normal hexane extracted substance in oil- and fat-containing wastewater flowing into the oil and fat decomposition vessel. By determining the amount of microbial formulation added in accordance with the amount of normal hexane extracted substance in oil- and fat-containing wastewater flowing into an oil and fat decomposition vessel in this manner, efficient decomposition of oil and fat can be achieved without expending microorganisms meaninglessly.

The oil- and fat-containing wastewater treatment method of the invention may comprise an additional step besides the steps described above. Examples of such a step include returning all or some of the water flowing out from an oil and fat decomposition vessel again to the oil and fat decomposition vessel. However, since the method of the invention can attain a sufficient effect of decomposing oil and fat without such a returning process, it is not required to return all or some of the water flowing out from an oil and fat decomposition vessel again to the oil and fat decomposition vessel.

Generally, microorganisms proliferate significantly while consuming the carbon source in a substrate in pure microorganism batch cultures. Typically, microorganisms proliferate about several hundred-fold. This was common general knowledge of bioengineering. However, even if there were oil and fat that can be the nutrient for added microorganisms, it is unlikely that microorganisms proliferate several hundred fold as in pure microorganism batch cultures in a flow of wastewater with infinite types and numbers of microorganisms. The microorganism concentration can even decease under conditions with an oil and fat concentration that is not that high.

However, efficient decomposition of oil and fat is possible even under such a condition with the present invention. The key of the present invention is not in how to make added microorganisms proliferate in wastewater. The present invention is based on a novel concept, which only requires added microorganism to be present in wastewater at a certain concentration or greater in accordance with the concentration of oil and fat.

With the method of the invention, the concentration of oil and fat can be effectively reduced, even for wastewater that continuously flows in and out and has various microorganisms, by continuously or intermittently adding a microbial formulation with an ability to decompose oil and fat to an oil and fat decomposition vessel. In the present invention, microorganisms do not necessarily need to proliferate in an oil and fat decomposition vessel. Oil and fat can be decomposed even if microorganism cannot proliferate.

The present invention is also a process in which it is difficult for added microorganisms to proliferate because various microorganisms are present other than the added microorganisms and oil- and fat-containing wastewater continuously flows in and out. Meanwhile, the present invention can establish the microorganisms in an oil and fat decomposition vessel without washout.

The method of the invention is compatible with a wide range of oil and fat concentrations, and is cost effective because an effect is attained with a lower amount of microorganisms than conventional art.

### (Apparatus and system)

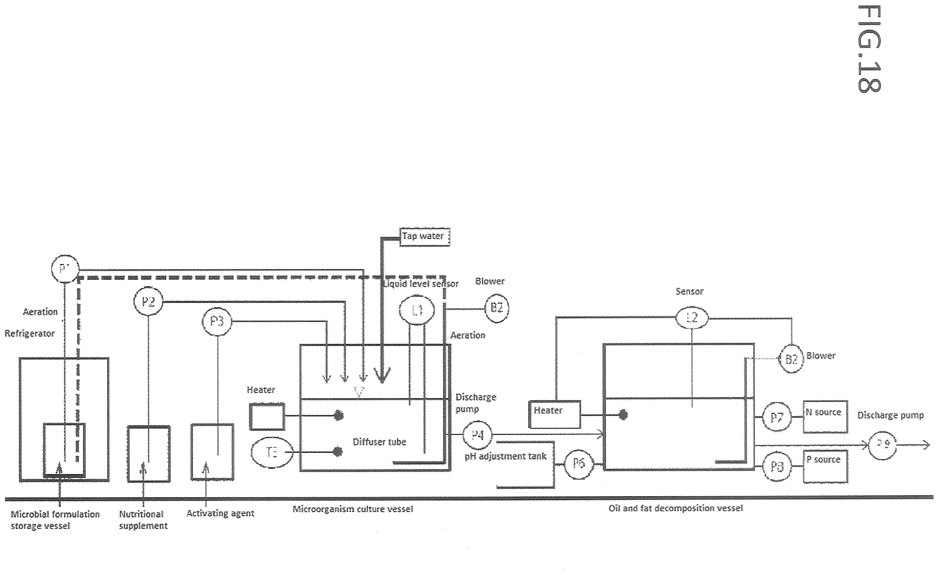

Also disclosed herein but not claimed is an apparatus or system for use in the treatment method of the invention. The apparatus or system may typically comprise a storage vessel for a microbial formulation, an amplification vessel for the microbial formulation, and an oil and fat decomposition vessel where oil- and fat-containing wastewater continuously flows in and out (Figure **18**).

The storage vessel for a microbial formulation in the apparatus or system is a vessel for storing a microbial formulation, which is a source of species transplantation, with a capacity of 30 to 200 L, but the capacity is not limited thereto. The storage vessel is typically made of, but is not limited to, metal, plastic, or glass. The storage vessel may comprise a blower for aeration as needed. In Figure **18****,** the storage vessel is stored in a refrigerator, but the configuration is not necessarily limited thereto.

In a preferred embodiment, the method of the invention uses a plurality of species of microorganisms as seed bacteria, and the storage vessel may comprise a plurality of storage tanks for storing various microorganisms separately by each type. The activity of microorganisms in a microbial formulation can be retained for an extended period of time by storing microorganisms separately by each type in this manner.

The amplification vessel for microorganisms may be typically a cylindrical or square-tubular and metal, plastic, or glass vessel with a volume of 30 to 1000 L. Preferably, an amplification vessel comprises a temperature controller and a thermometer and can control the temperature during culture (amplification) of microorganisms. The controlled temperature is typically 18 to 35°C, preferably 25 to 33°C, and more preferably 28 to 30°C. A water level gauge or a liquid level sensor **L1** may be installed in an amplification vessel to enable controlling of the liquid level to a lower limit, upper limit, or middle level. Furthermore, such a liquid level sensor may also function as a foaming sensor, or a system for sensing foaming by another principle may be installed. The vessel may comprise a system for automatically supplying a defoamer that is interlinked with such a system for sensing foaming. An amplification vessel may also comprise a blower.

A specified amount of a microbial formulation stored in a storage vessel, a nutritional supplement, and an activating agent may be automatically supplied to the amplification vessel through pump **P1,** pump **P2,** and pump **P3,** respectively.

An activating agent is stored in an activating agent tank. An activating agent tank is typically a metal, plastic, or glass tank, preferably a plastic tank, with a capacity of typically 3 to 100 L and preferably 10 to 50 L, for storing an inorganic salt solution comprising a nutrient required for proliferation of microorganisms. An activating agent tank may be stored in a refrigerator.

A nutritional supplement is stored in a nutritional supplement tank. This is typically a metal, plastic, or glass tank, preferably a plastic tank, with a capacity of typically 3 to 100 L and preferably 10 to 50 L, for storing oil used as a carbon source of microorganisms. A nutritional supplement tank may be stored in a refrigerator.

While not shown, the system may further comprise tank with a capacity of typically 3 to 100 L and preferably 10 to 50 L for storing an organic matter other than oil used as a carbon source or microorganisms, or a solution thereof. This is typically a metal, plastic, or glass tank, and preferably a plastic tank. This tank may be stored in a refrigerator.

A diffuser tube, spherical air diffuser, or microbubble/nanobubble generator may be installed in a culture vessel and a formulation tank.

Microorganisms that have proliferated in a microorganism culture vessel are added to the oil and fat decomposition vessel via pump **P4,** and oil and fat in wastewater is decomposed in the oil and fat decomposition vessel. The oil and fat decomposition vessel may comprise a tank, a blower, and a sensor **L2.** The sensor **L2** of the oil and fat decomposition vessel may be a sensor for detecting one or more of a temperature, a pH, a dissolved oxygen concentration, and a foaming level.

The apparatus or system may also comprise an execution/control unit storing a computer program (also simply referred to as "program") for executing the oil- and fat-containing wastewater treatment method of the invention and/or a program for controlling the apparatus or system. There is also disclosed herein a recording medium (e.g., CD-R, DVD, USB (flash) memory, or the like) for storing a program for executing the oil- and fat-containing wastewater treatment method of the invention and/or a program for controlling the apparatus or system.

For example, such a program may be coded to make the apparatus or system execute the step of decomposing oil and fat by continuously or intermittently adding a microbial formulation with an ability to decompose oil and fat to an oil and fat decomposition vessel where oil- and fat-containing wastewater continuously flows in and out, or to implement the control of the apparatus or system. The execution/control unit or program used is configured to calculate and/or control the amount of added microbial formulation to be used to be the amount at which a microorganism can be established in an oil and fat decomposition vessel. The execution/control unit or program may be configured to implement controlling of the apparatus or system, so that the amount of microbial formulation added is an amount resulting in 5.0 × 10⁴ to 1.0 × 10⁷ cells/mL with respect to wastewater in the oil and fat decomposition vessel, or the amount is for example, 3.0 × 10⁴ to 1.0 × 10⁸ cells and preferably 3.0 × 10⁴ to 1.0 × 10⁷ cells per 1 mg of normal hexane extracted substance in oil- and fat-containing wastewater flowing into the oil and fat decomposition vessel. The amount added may be appropriately changed depending on the actual circumstance of use, based on the descriptions herein. The apparatus or system may also be configured to be able to measure the volume of wastewater or a value related to normal hexane extracted substance in the wastewater to determine the amount added.

Some or all of the various functions materialized by the execution/control unit or program may be materialized manually.

Some or all of the various functions materialized by the execution/control unit or program may be materialized or optimized by artificial intelligence (AI) or machine learning.

The apparatus or system can be configured to be capable of controlling the dissolved oxygen concentration of oil- and fat-containing wastewater in an oil and fat decomposition vessel. Such a configuration may be materialized by separately providing an oxygen concentration control unit for controlling the oxygen concentration or by controlling the concentration using the execution/control unit or program described above. Some or all of the configuration may be materialized manually, or materialized or optimized by artificial intelligence (AI) or machine learning. While the oxygen concentration is supplied using an air supply source such as a blower or a compressor, the amount supplied is automatically adjusted in concert with the dissolved oxygen concentration. Furthermore, in order to increase the dissolution efficiency of oxygen, an agitator or the like can be provided, and the agitation rate can be controlled to move in concert with the dissolved oxygen concentration. Furthermore, a system can be incorporated, which indirectly monitors the decomposition activity based on the variation in dissolved oxygen, determines that decomposition of oil has not been completed or progressed when dissolved oxygen hardly decreases, and feeds back and control the amount of microbial formulation added.

The apparatus or system may also be configured to control the pH of oil- and fat-containing wastewater in an oil and fat decomposition vessel. Such a configuration may be materialized by separately providing a pH control unit for controlling the pH or by controlling pH using the execution/control unit or program described above. Some or all of the configuration may be materialized manually, or materialized or optimized by artificial intelligence (AI) or machine learning. pH control may be materialized by any means or approach that is known in the art, such as adding an appropriate amount of acid or alkali. Therefore, pH control may be materialized by measuring the pH, calculating the amount of acid and/or alkali to be added based on the measurement value or other parameters (e.g., volume or the like), and adding acid and/or alkali based on the result of calculation. For example, an oil and fat decomposition vessel may comprise a pH adjustment tank for automatically adjusting the pH when the pH is beyond the specified range, as shown in Figure **18****.** A pH adjustment tank may automatically adjust the pH in an oil and fat decomposition vessel to within the specified range of pH, when the pH is beyond the specified range, by automatically adding to the oil and fat decomposition vessel an aqueous solution of 2N sulfuric acid or 2N sodium hydroxide or the like via, for example, pump **P6.** Alternatively, ammonium or an aqueous solution thereof may be added instead of sodium hydroxide to simultaneously supply a nitrogen source. Furthermore, a system may be incorporated, which indirectly monitors the decomposition activity based on the decrease in pH and the amount of alkali consumption by utilizing the tendency for treated water in a vessel to be acidic with the progression of decomposition of oil and fat, and feeds back and controls the amount of microbial formulation added.

The apparatus or system may be configured to control the temperature of oil- and fat-containing wastewater in an oil and fat decomposition vessel. Such a configuration may be materialized by separately providing a temperature control unit, or temperature control may be materialized by the execution/control unit or program described above. Some or all of the configuration may be materialized manually, or materialized or optimized by artificial intelligence (AI) or machine learning. Such temperature control preferably comprises, but is not limited to, the ability to control the temperature within the range of 12 to 42°C. Temperature control can be achieved by any means that is known in the art. For example, wastewater can be heated by a heater when the temperature measured at sensor **L2** is below the specified range to increase the temperature to the specified range, as shown in Figure **18****.** Alternatively, waste heat such as waste heat of a boiler, direct addition of high temperature wastewater, or heat exchange may be utilized. If the temperature measured at sensor **L2** exceeds the specified temperature range, wastewater can be cooled by a cooler to reduce the temperature to the specified temperature. Alternatively, infusion of cooling water, heat exchange with cold water, or supply of cold air may be utilized. Furthermore, a system may be incorporated, which senses the extent of increase in the temperature or the operation of the cooling system by utilizing the principle of increase in temperature by heat produced from fermentation due to decomposition, and feeds back and controls the amount of microbial formulation added.

The apparatus or system may be configured to control C/N of oil- and fat-containing wastewater in an oil and fat decomposition vessel. The range of C/N of the oil- and fat-containing wastewater is preferably, but is not limited to, 2 to 50, 2 to 30, or 2 to 20. Such C/N control may be materialized by separately providing a C/N control unit, or by controlling C/N with the execution/control unit or program described above. Some or all of the configuration may be materialized manually, or materialized or optimized by artificial intelligence (AI) or machine learning. If C/N is not within the range described above due to insufficient nitrogen in wastewater, a required amount of nitrogen (preferably ammonium sulfate or urea) may be added, for example, through pump **P7** from an N source tank (Figure **18**). Alternatively, ammonium water or the like may be added in a form that is interlinked with pH adjustment.

The apparatus or system may also be configured to control N/P of oil- and fat-containing wastewater in an oil and fat decomposition vessel. The range of N/P of the oil- and fat-containing wastewater is preferably, but is not limited to, 1 to 20, 1 to 10, or 1 to 5. Such N/P control may be materialized by separately providing an N/P control unit, or by controlling N/P with the execution/control unit or program described above. Some or all of the configuration may be materialized manually, or materialized or optimized by artificial intelligence (AI) or machine learning. If N/P is not within the range described above due to insufficient phosphorous in wastewater, a required amount of phosphorous, in a form of phosphate, may be added, for example, through pump **P8** from a P source tank (Figure **18**). Further, an aqueous solution comprising phosphate and the nitrogen source described above may be supplied together after storage in a single tank as a single activating agent that is prepared in advanced so that N/P would be in the range described above.

The apparatus or system may be configured to control the amount of added microorganisms in water flowing out from an oil and fat decomposition vessel. Such controlling of the amount of added microorganisms may be materialized by separately providing an added microorganism amount control unit, or by control said amount with the execution/control unit or program described above. Some or all of the configuration may be materialized manually, or materialized or optimized by artificial intelligence (AI) or machine learning.

For example, an oil and fat decomposition vessel may also comprise a foaming sensor for detecting the status of foaming. The oil and fat decomposition vessel may further comprise a defoaming tank that automatically adds a defoamer when foaming is detected by a foaming sensor. Furthermore, foaming often indicates active decomposition of oil and fat. Thus, a system may be incorporated, which utilizes this concept, and feeds back and controls the amount of microbial formulation added in concert with a foaming sensor or addition of a defoamer.

The oil and fat decomposition vessel may be further equipped with a water sampling pump for sampling a required amount at a designated date and time. A liquid may be sent to a water sampling tank within a refrigerator in accordance with the setting.

The oil and fat decomposition vessel may comprise a determination means for determining the extent of decomposition of oil and fat in wastewater in the oil and fat decomposition vessel. The determination through the determination means may be, for example, centrifuging the wastewater and evaluating the extent of decomposition of oil and fat from the status of the supernatant thereof.

Wastewater treated in an oil and fat decomposition vessel may be subjected to later stage treatment in an activated sludge vessel or the like through, for example, pump **P9.**

### [Examples]

Examples are provided hereinafter to describe the present invention in more detail. However, the present invention is not limited whatsoever to these Examples or the like.

### Test Example 1: Effect of dissolved oxygen concentration (DO)

7L of food plant wastewater comprising oil content with an n-Hex value of about 10000 mg/L was loaded into a 10 L fermenter (Biott Co., Ltd), and decomposition tests were conducted on oil content at 30°C in batches under various dissolved oxygen concentrations. Colony counting in an LB agar medium showed that the wastewater initially contained microorganisms at about 3 × 10⁸ cells/mL. A mixed formulation of Burkholderia arboris and Yarrowia lipolytica was used as a microbial formulation. Bacteria were seeded at the start of the decomposition test so that the microorganism concentration of the former would be 1.0 × 10⁶ cells/mL. Since the microorganism concentration of the latter is generally about 1/10 of the cell count of the former upon seeding and during culture, the concentration of the former was used as the concentration of the total added microorganisms.

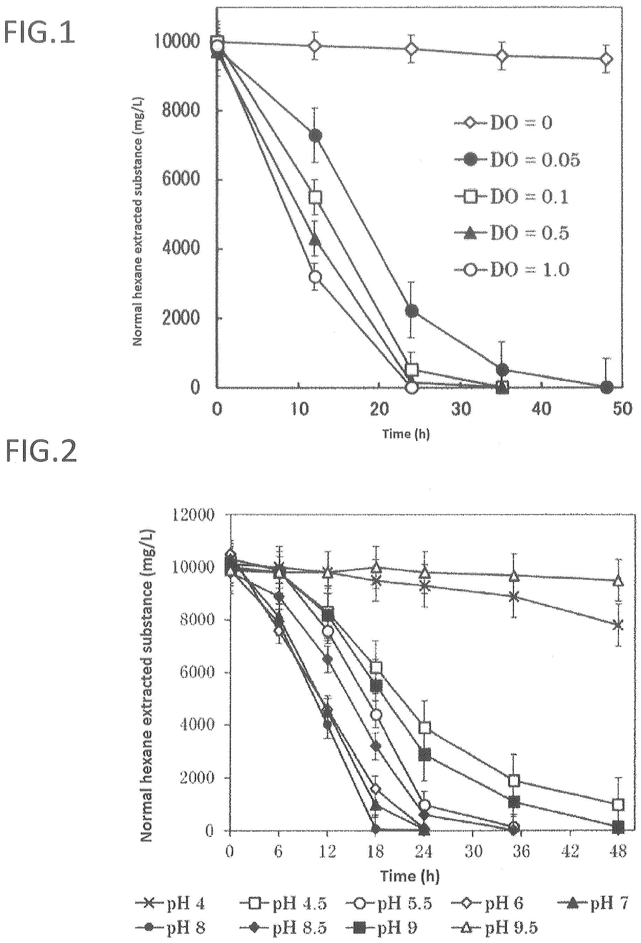

The dissolved oxygen concentration (DO) was constantly monitored with a galvanic DO electrode for fermentation and adjusted with the amount of aeration and agitation rate. During the decomposition test, pH was constantly monitored with a sealed pH electrode and automatically adjusted by adding dilute hydrochloric acid and aqueous sodium hydroxide solution so that the pH would be 6.0 to 8.0. The test solution was chronologically sampled. The n-Hex extracted substance concentration was measured in accordance with JIS K 0102. Batch testing was performed three times. The standard error thereof is denoted in the graph. The results are shown in Figure **1****.**

### Test Example 2: Effect of pH

The same 7L of food plant wastewater as that in Test Example 1 was loaded into a 10 L fermenter (Biott Co., Ltd), and decomposition tests were conducted on oil content at 30°C in batches under various pH conditions. The same microbial formulation as that in Test Example 1 was used. Bacteria were seeded at the start of the decomposition test so that the Burkholderia arboris concentration would be 1.0 × 10⁶ cells/mL. During the decomposition tests, the samples were aerated and agitated so that the DO would be 1.0 or greater. DO and pH were measured and adjusted by the same methods as those in Test Example 1. The test solution was chronologically sampled. The n-Hex extracted substance concentration was measured by the same method as that in Test Example 1. Batch testing was performed three times. The standard error thereof is denoted in the graph. The results are shown in Figure **2****.**

### Test Example 3: Effect of temperature

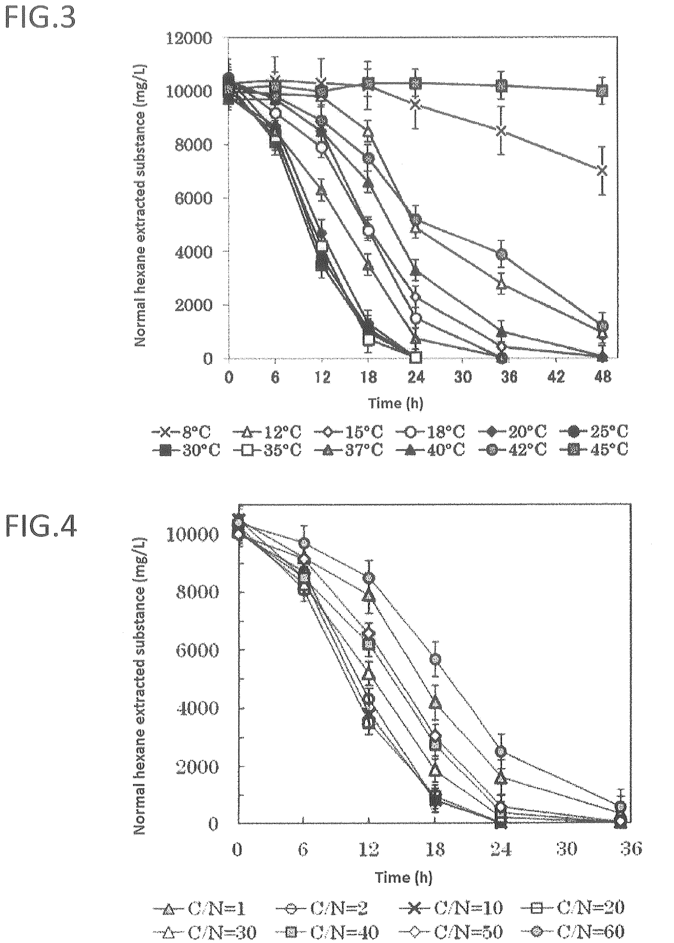

The same 7L of food plant wastewater as that in Test Example 1 was loaded into a 10 L fermenter (Biott Co., Ltd), and decomposition tests were conducted on oil content in batches under various temperature conditions. The same microbial formulation as that in Test Example 1 was used. Bacteria were seeded at the start of the decomposition test so that the Burkholderia arboris concentration would be 1.0 × 10⁶ cells/mL. During the decomposition tests, the samples were aerated and agitated so that the DO would be 1.0 or greater, and the pH was adjusted to be 6.0 to 8.0. DO and pH were measured and adjusted by the same methods as those in Test Example 1. The test solution was chronologically sampled. The n-Hex extracted substance concentration was measured by the same method as that in Test Example 1. Batch testing was performed three times. The standard error thereof is denoted in the graph. The results are shown in Figure **3****.**

### Test Example 4: Effect of the ratio of carbon to nitrogen (C/N)

7L of inorganic salt medium comprising 1% canola oil (corresponding to n-hex of about 10000 mg/L) was loaded into a 10 L fermenter (Biott Co., Ltd), and decomposition tests were conducted on oil content at 30°C and pH of 6.0 to 8.0 in batches under various C/N (weight ratio of each element) conditions. The composition of the inorganic salt medium was 3.5 g/L of Na₂HPO₄, 2.0 g/L of KH₂PO₄, 0.775 to 3.6 g/L of (NH₄)₂SO₄, 0.34 g/L of MgCl₂·6H₂O, 2.8 mg/L of FeSO₄7H₂O, 2.4 mg/L of MnSO₄·5H₂O, 2.4 mg/L of CoCl₂·6H₂O, 1.7 mg/L of CaCl₂·2H₂O, 0.2 mg/L of CuCl₂·2H₂O, 0.3 mg/L of ZnSO₄·7H₂O, and 0.25 mg/L of NaMoO₄. C/N of 10 or greater was adjusted by changing the concentration of ammonium sulfate ((NH₄)₂SO₄). C/N of 2 or less was adjusted by adding urea thereto.

The carbon content of canola oil was calculated in units of trioleate. 1 L of activated sludge at a sewage treatment plant known to have an n-Hex value of 100 mg/L or less that was centrifuged and resuspended into 50 mL of the inorganic salt medium described above was added thereto. As a result, a test solution which already had about 5.0 × 10⁶ cells/mL of culturable microorganisms was prepared. The same microbial formulation as that in Test Example 1 was seeded thereto at the start of the decomposition test so that the Burkholderia arboris concentration would be 1.0 × 10⁶ cells/mL. During the decomposition tests, the samples were aerated and agitated so that the DO would be 1.0 or greater. DO and pH were measured and adjusted by the same methods as those in Test Example 1. The test solution was chronologically sampled. The n-Hex extracted substance concentration was measured in accordance with JIS K 0102. Batch testing was performed three times. The standard error thereof is denoted in the graph. The results are shown in Figure **4****.**

### Test Example 5: Effect of the ratio of nitrogen to phosphorous (N/P)

7L of inorganic salt medium comprising 1% canola oil (corresponding to n-hex of about 10000 mg/L) was loaded into a 10 L fermenter (Biott Co., Ltd), and decomposition tests were conducted on oil content at 30°C and pH of 6.0 to 8.0 in batches under various N/P (weight ratio of each element) conditions. The composition of the inorganic salt medium was the same as that in Test Example 4, except for the concentration of ammonium sulfate and phosphate. The concentration of (NH₄)₂SO₄, i.e., the nitrogen source, was fixed at 4 g/L, and N/P was adjusted by changing the concentration of phosphorous sources Na₂HPO₄ and KH₂PO₄. However, Na₂HPO₄:KH₂PO₄ (weight ratio) was fixed to 3.5:2.

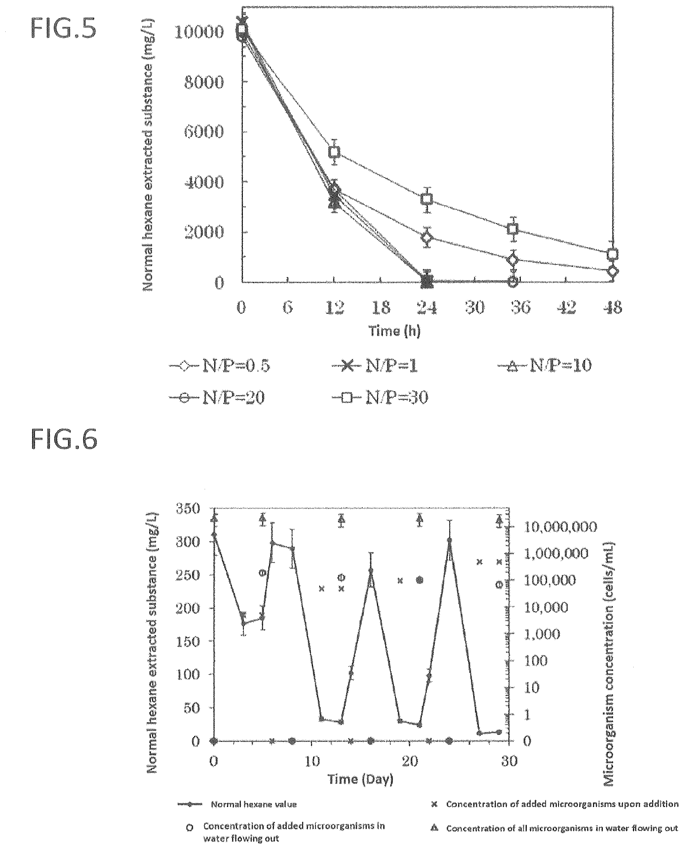

The same microbial formulation as that in Test Example 1 was used and seeded at the start of the decomposition test so that the Burkholderia arboris concentration would be 1.0 × 10⁶ cells/mL. During the decomposition tests, the samples were aerated and agitated so that the DO would be 1.0 or greater. DO and pH were measured and adjusted by the same methods as those in Test Example 1. The test solution was chronologically sampled. The n-Hex extracted substance concentration was measured in accordance with JIS K 0102. Batch testing was performed three times. The standard error thereof is denoted in the graph. The results are shown in Figure **5****.**

### Test Example 6: Effect of the concentration of added microorganisms upon addition by intermittent addition

An independently designed and manufactured site verification demo version tester was installed in a wastewater treatment site at a factory. The demo version tester consists of a raw water relay vessel (50 L), oil decomposition vessel (60 L), activated sludge vessel (30 L), precipitation vessel (12.5 L), and discharge relay vessel (50 L). Wastewater at the site is drawn into the raw water relay vessel through a pump and sequentially flows through the oil decomposition vessel, activated sludge vessel, and precipitation vessel continuously. Each of these vessels is coupled to the discharge relay vessel, so that any amount can be directly discharged from each vessel. The treatment volume and retention time can be set for each vessel. Any amount of precipitated sludge can be returned to the activated sludge vessel from the precipitation vessel.

The temperature, pH, DO, and MLSS are constantly monitored with an installed sensor in the oil decomposition vessel. The foaming status is also constantly monitored with a foaming sensor. When foaming is detected, a defoamer is automatically added. The pH is adjusted by automatically adding an aqueous solution of 2N sulfuric acid or 2N sodium hydroxide from a pH adjustment tank when the pH is not within the specified range. Aeration is performed by supplying air from a blower in the amount that is adjusted with a flowmeter in an oil decomposition vessel or activated sludge vessel. The oil decomposition vessel is supplied with a microbial formulation from a microbial formulation tank installed in a refrigerator through a pump at a specified volume and time. Furthermore, a water sampling pump is installed in the oil decomposition vessel so that a required amount can be sampled at a designated time and date. A liquid is sent to a water sampling tank in a refrigerator in accordance with the setting.

A decomposition test with intermittent addition of a microbial formulation was conducted by allowing actual wastewater to continuously flow in the demo version tester. The microorganism concentration upon intermittent addition was increased stepwise at the site. The same mixed formulation of Burkholderia arboris and Yarrowia lipolytica as that in Test Example 1 was used as a microbial formulation. The effect of decomposing and removing oil content was studied by measuring the concentration of n-Hex extracted substance in accordance with JIS K 0102 for samples of water flowing out from the oil and fat decomposition vessel immediately after the retention time on day 3 and day 5 when data stabilizes for the concentration of added microorganism upon each addition. Since this resulted in taking measurements twice each (day 3 and day 5) under the same conditions, reproducibility was confirmed.

Furthermore, the microorganism concentration in water flowing out was measured to investigate the status of proliferation of added microorganisms upon sampling on day 5 (o symbol in the graph). The microorganism concentration was measured by real-time PCR targeting 16S ribosome DNA of Burkholderia arboris for the added microorganisms, and by colony counting in the LB medium for all microorganisms. Real-time PCR was performed three times for each sample. The all microorganism concentration was measured by preparing three plates for each dilution level and finding the standard error. Before increasing the microorganism concentration, supply of microorganisms was temporarily discontinued for three days to investigate the effect on the n-Hex value. Under each condition, the pH in the oil and fat decomposition vessel was automatically adjusted to the vicinity of neutral. The water temperature was between 25 to 35°C. During the treatment, the samples were aerated and agitated so that DO would be 1 mg/L or greater. The n-Hex value was measured three times for each sample in accordance with JIS K 0102, and the standard error was found.

### 6-1. Results in low concentration oil and fat (n-Hex value of about 300 mg/L) wastewater

At a site with an n-Hex value level of 300 mg/L and wastewater volume of 100 ton/day, tests were conducted at a 1/1000 scale at a treatment rate of 100 L/day. Water volume to be treated in the oil and fat decomposition vessel was set to 50 L, and the retention time was set to 12 hours. Microbial formulations with a concentration of 5 × 10⁶, 5 × 10⁷, 1 × 10⁸, or 5 × 10⁸ cells/mL were added twice a day to correspond to the retention time at 50 mL each, which is 1/1000 of the water volume to be treated. The results are shown in Figure **6****.** The concentrations of added microorganisms upon addition were 5 × 10³, 5 × 10⁴, 1 × 10⁵, and 5 × 10⁵ cells/mL (× symbol).

As a result, the n-Hex value dramatically decreased at a concentration of added oil decomposing bacteria upon addition of 5 × 10⁴ cells/mL or greater. When addition of microorganisms was discontinued (concentration of added microorganisms upon addition of 0), the concentration of oil content increased on the same day, but decomposition of oil content quickly recovered by re-addition. Despite oil and fat being decomposed, 100-fold or greater proliferation in added microorganisms was not observed at any of the amounts added. Further, proliferation of the microorganisms was not observed at a concentration of added oil decomposing bacteria upon addition of 1 × 10⁵ cells/mL, and decreased at 5 × 10⁵ cells/mL.

While attaining an oil and fat decomposition effect without proliferation of microorganisms in culture is inconsistent with the common general knowledge of bioengineering, the cause is understood to be insufficient amount of carbon source for proliferation in view of the oil and fat concentration that is not particularly high. It can be understood that oil was sufficiently decomposed by microorganisms at the added concentration without proliferation. Thus, it was found that addition of a high concentration of microorganism beyond what is required is meaningless.

The wastewater initially contained about 2 × 10⁷ cells/mL of microorganisms (Δ symbol in Figure **6**) besides the added microorganisms. There was no significant change in the concentration of all microorganism after addition of an oil and fat decomposing microbial formulation. The concentration of added microorganisms in water flowing out was 1% or less of the concentration of all microorganisms, such that the effect of decomposition can be exerted without the added microorganisms becoming the dominant or primary species. This is also inconsistent with conventional common general knowledge of bioengineering.

### 6-2. Results in moderate concentration oil and fat (n-Hex value of about 3000 mg/L) wastewater

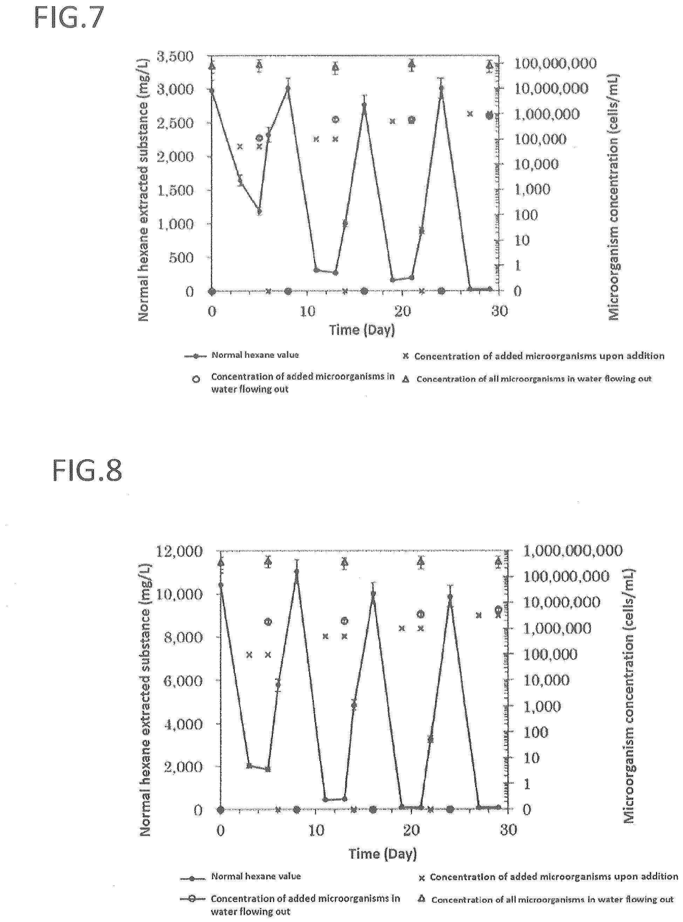

At a site with an n-Hex value level of 3000 mg/L and wastewater volume of 100 ton/day, tests were conducted at a 1/1250 scale at a treatment rate of 80 L/day. Water volume to be treated in the oil and fat decomposition vessel was set to 60 L, and the retention time was set to 18 hours. Microbial formulations with a concentration of 5 × 10⁷, 1 × 10⁸, 5 × 10⁸, or 1 × 10⁹ cells/mL were added every 18 hours to correspond to the retention time at 60 mL each. The results are shown in Figure **7****.** The concentrations of added microorganisms upon addition were 5 × 10⁴, 1 × 10⁵, 5 × 10⁵, and 1 × 10⁶ cells/mL (× symbol).

As a result, the n-Hex value dramatically decreased at a concentration of added microorganisms upon addition of 1 × 10⁵ cells/mL or greater. When addition of microorganisms was discontinued (concentration of added microorganisms upon addition of 0), the concentration of oil content increased on the same day, but decomposition of oil content quickly recovered by re-addition. Unlike normal microorganism culture, significant proliferation of added microorganisms was not found even if oil content was effectively decomposed, and the microorganism concentration rather decreased when the added oil decomposing bacterial concentration upon addition was 1 × 10⁶ cells/mL. The cause is understood to be insufficient amount of carbon source for proliferation in view of the oil and fat concentration that is not particularly high. It can be understood that oil content was sufficiently decomposed by microorganisms at the added concentration without proliferation. Thus, it was found that addition of a high concentration of microorganism beyond what is required is meaningless.

The wastewater initially contained about 8 × 10⁷ cells/mL of microorganisms (Δ symbol in Figure **7**) besides the added microorganisms. There was no significant change in the concentration of all microorganisms after addition of an oil and fat decomposing microbial formulation. The concentration of added microorganisms in water flowing out was about 1% or less of the concentration of all microorganisms, such that the effect of decomposition was sufficiently exerted without the added microorganisms becoming the dominant or primary species.

### 6-3. Results in high concentration oil and fat (n-Hex value of about 10000 mg/L) wastewater

At a site with an n-Hex value level of 30000 mg/L and wastewater volume of 100 ton/day, wastewater was diluted 3-fold with groundwater to prepare a diluted wastewater with an n-Hex value of about 10000 mg/L, and tests were conducted at a 1/1250 scale at a treatment rate of 80 L/day. Water volume to be treated in the oil and fat decomposition vessel was set to 60 L, and the retention time was set to 18 hours. Microbial formulations with a concentration of 1 × 10⁸, 5 × 10⁸, 1 × 10⁹, or 3 × 10⁹ cells/mL were added every 18 hours to correspond to the retention time at 60 mL each. The results are shown in Figure **8****.** The concentrations of added microorganisms upon addition were 1 × 10⁵, 5 × 10⁵, 1 × 10⁶, and 3 × 10⁶ cells/mL (× symbol).

As a result, the n-Hex value decreased 95% or more at a concentration of added microorganisms upon addition of 5 × 10⁵ cells/mL or greater. When addition of microorganisms was discontinued (concentration of added microorganisms upon addition of 0), the concentration of oil content increased on the same day, but decomposition of oil content quickly recovered by re-addition. Unlike normal microorganism culture, 100-fold or greater proliferation of added microorganisms was not found at any of the amounts added, despite oil and fat being decomposed. Furthermore, 10-fold or greater proliferation of added microorganisms was not found, even if oil content was very effectively decomposed. At a concentration of added microorganisms upon addition of 1 × 10⁶ cells/mL, a sufficient effect of oil decomposition and removal was attained. Significance in adding more microorganisms was hardly found.

The diluted wastewater initially contained about 4 × 10⁸ cells/mL of microorganisms (Δ symbol in Figure **8**) besides the added microorganisms. There was no significant change in the concentration of all microorganisms after addition of an oil and fat decomposing microbial formulation. The concentration of added microorganisms in water flowing out was about 1% or less of the concentration of all microorganisms, such that the effect of decomposition was sufficiently exerted without the added microorganisms becoming the dominant or primary species.

### 6-4. Results in ultrahigh concentration oil and fat (n-Hex value of about 30000 mg/L) wastewater

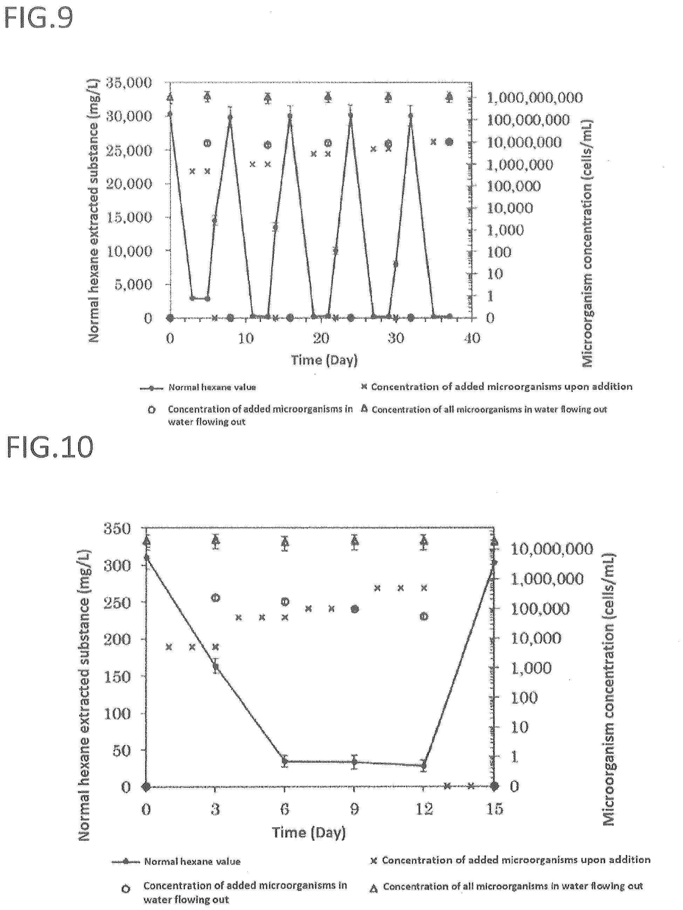

At a site with an n-Hex value level of 30000 mg/L and wastewater volume of 100 ton/day, tests were conducted at a 1/2000 scale at a treatment rate of 50 L/day. Water volume to be treated in the oil and fat decomposition vessel was set to 50 L, and the retention time was set to 24 hours. Microbial formulations with a concentration of 5 × 10⁸, 1 × 10⁹, 3 × 10⁹, 5 × 10⁹, and 1 × 10¹⁰ cells/mL were added every 24 hours to correspond to the retention time at 50 mL each. The results are shown in Figure **9****.** The concentrations of added microorganisms upon addition were 5 × 10⁵, 1 × 10⁶, 3 × 10⁶, 5 × 10⁶, and 1 × 10⁷, cells/mL (× symbol).

As a result, the n-Hex value decreased 99% or more at a concentration of added microorganisms upon addition of 1 × 10⁶ cells/mL or greater. When addition of microorganisms was discontinued (concentration of added microorganisms upon addition of 0), the concentration of oil content increased on the same day, but decomposition of oil content quickly recovered by re-addition. Unlike normal microorganism culture, 100-fold or greater proliferation of added microorganisms was not found at any of the amounts added, despite oil and fat being decomposed. Furthermore, 10-fold or greater proliferation of added microorganisms was not found, even if oil content was very effectively decomposed. The level of proliferation of microorganisms was similar even if the concentration of added microorganisms was changed. The proliferation was at most to the level of 1 × 10⁷ cells/mL. Thus, any more addition of microorganisms would be meaningless.

The wastewater initially contained about 1 × 10⁹ cells/mL or more of microorganisms (Δ symbol in Figure **9**) besides the added microorganisms. There was no significant change in the concentration of all microorganisms after addition of an oil and fat decomposing microbial formulation. The concentration of added microorganisms in water flowing out was 1% or less of the concentration of all microorganisms, such that the effect of decomposition was sufficiently exerted without the added microorganisms becoming the dominant or primary species.

### Test Example 7: Effect of the concentration of added microorganisms upon addition in continuous addition

The same site verification demo version tester as that in Test Example 6 was installed in a wastewater treatment site at a factory. Decomposition tests that continuously add a microbial formulation while allowing wastewater to continuously flow were conducted. The supplied microorganism concentration at each site was increased stepwise every three days, so that the concentration of added microorganisms upon addition was within the range of 5 × 10³ to 1 × 10⁷ cells/mL. The same mixed formulation of Burkholderia arboris and Yarrowia lipolytica as that in Test Example 1 was used as a microbial formulation. The microorganism concentration of the former was used as the concentration of total added microorganisms.

The water flowing out was sampled on day 3 for the concentration of each added microorganism, and the concentration of n-Hex extracted substance was measured in accordance with JIS K0102 to investigate the effect of decomposing and removing oil content. The concentrations of added microorganisms and all microorganisms in water flowing out were analyzed by the same approach as that in Test Example 6. Lastly, supply of microbial formulation was discontinued to investigate the effect on the n-Hex value. Under each condition, the pH in the oil and fat decomposition vessel was automatically adjusted to the vicinity of neutral. The water temperature was between 25 to 35°C. During the treatment, the samples were aerated and agitated so that DO would be 1 mg/L or greater. The n-Hex value was analyzed three times for each sample, and the standard error was found.

### 7-1. Results in low concentration oil and fat (n-Hex value of about 300 mg/L) wastewater

At a site with an n-Hex value level of 300 mg/L and wastewater volume of 100 ton/day, tests were conducted at a 1/1000 scale at a treatment rate of 100 L/day. Water volume to be treated in the oil and fat decomposition vessel was set to 50 L, and the retention time was set to 12 hours. Microbial formulations with a concentration of 5 × 10⁶, 5 × 10⁷, 1 × 10⁸, or 5 × 10⁹ cells/mL were added continuously at a supply rate of 4.17 mL/h, which is 1/1000 of the water volume to be treated. The results are shown in Figure **10****.** The concentrations of added microorganisms upon addition were 5 × 10³, 5 × 10⁴, 1 × 10⁵, and 5 × 10⁵ cells/mL (× symbol).

As a result, in the same manner as intermittent addition, the n-Hex value dramatically decreased at a concentration of added microorganisms upon addition of 5 × 10⁴ cells/mL or greater. When addition of microorganisms was discontinued (concentration of added microorganisms upon addition of 0), the concentration of oil content increased on the same day. Despite oil and fat being decomposed, 100-fold or greater proliferation of added microorganisms was not observed at any of the amounts added. Further, proliferation of microorganisms was not observed at a concentration of added microorganisms upon addition of 1 × 10⁵ cells/mL, and microorganisms decreased at 5 × 10⁵ cells/mL. It was found that addition of a high concentration of microorganism beyond what is required is meaningless.

The wastewater initially contained about 2 × 10⁷ cells/mL of microorganisms (Δ symbol in Figure **10**) besides the added microorganisms. There was no significant change in the concentration of all microorganism after addition of an oil and fat decomposing microbial formulation. The concentration of added microorganisms in water flowing out was about 1% or less of the concentration of all microorganisms, such that the effect of decomposition was able to be exerted without the added microorganisms becoming the dominant or primary species.

### 7-2. Results in moderate concentration oil and fat (n-Hex value of about 3000 mg/L) wastewater

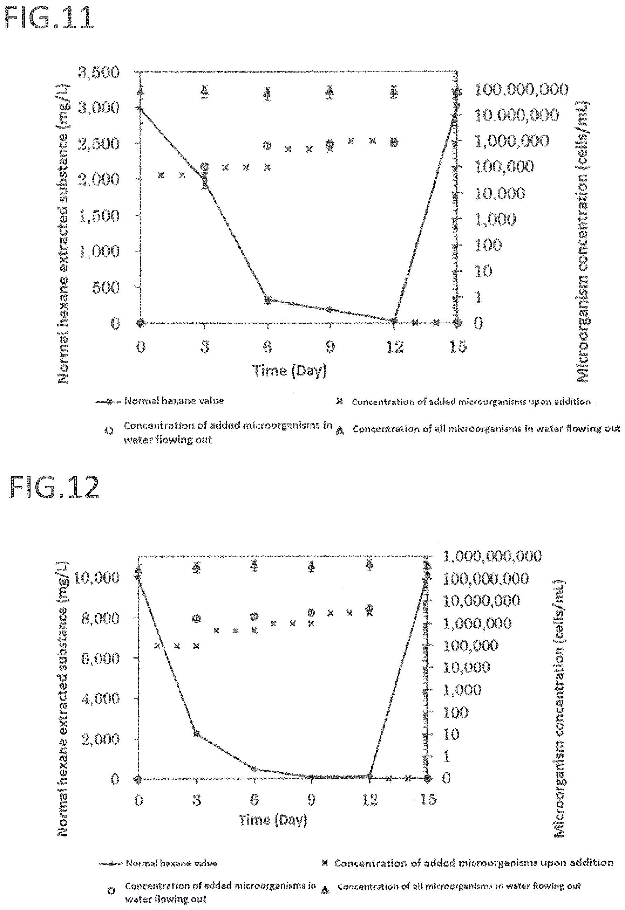

At a site with an n-Hex value level of 3000 mg/L and wastewater volume of 100 ton/day, tests were conducted at a 1/1250 scale at a treatment rate of 80 L/day. Water volume to be treated in the oil and fat decomposition vessel was set to 60 L, and the retention time was set to 18 hours. Microbial formulations with a concentration of 5 × 10⁷, 1 × 10⁸, 5 × 10⁸, or 1 × 10⁹ cells/mL were added continuously at a supply rate of 3.3 mL/h, which is 1/1000 of the water volume to be treated. The results are shown in Figure 11. The concentrations of added microorganisms upon addition were 5 × 10⁴, 1 × 10⁵, 5 × 10⁵, and 1 × 10⁶ cells/mL (× symbol).

As a result, the n-Hex value dramatically decreased at a concentration of added microorganisms upon addition of 1 × 10⁵ cells/mL or greater, in the same manner as the intermittent addition. When addition of microorganisms was discontinued (concentration of added microorganisms upon addition of 0), the concentration of oil content increased on the same day. Significant proliferation of added microorganisms was not found, even if oil content was effectively decomposed. In contrast, the microorganisms did not proliferate at all when the concentration of added microorganisms upon addition was 1 × 10⁶ cells/mL. Thus, it was found that addition of a high concentration of microorganism beyond what is required is meaningless.

The wastewater initially contained about 8 × 10⁷ cells/mL of microorganisms (Δ symbol in Figure 11) besides the added microorganisms. There was no significant change in the concentration of all microorganisms after addition of an oil and fat decomposing microbial formulation. The concentration of added microorganisms in water flowing out was 1% or less of the concentration of all microorganisms, such that the effect of decomposition was sufficiently exerted without the added microorganisms becoming the dominant or primary species.

### 7-3. Results in high concentration oil and fat (n-Hex value of about 10000 mg/L) wastewater

At a site with an n-Hex value level of 30000 mg/L and wastewater volume of 100 ton/day, wastewater was diluted 3-fold with groundwater to prepare a diluted wastewater with an n-Hex value of about 10000 mg/L, and tests were conducted at a 1/1250 scale at a treatment rate of 80 L/day. Water volume to be treated in the oil and fat decomposition vessel was set to 60 L, and the retention time was set to 18 hours. Microbial formulations with a concentration of 1 × 10⁸, 5 × 10⁸, 1 × 10⁹, or 3 × 10⁹ cells/mL were added continuously at a supply rate of 3.3 mL/h, which is 1/1000 of the water volume to be treated. The results are shown in Figure **12****.** The concentrations of added microorganisms upon addition were 1 × 10⁵, 5 × 10⁵, 1 × 10⁶, and 3 × 10⁶ cells/mL (× symbol).

As a result, in the same manner as intermittent addition, the n-Hex value decreased 95% or more at a concentration of added microorganisms upon addition of 5 × 10⁵ cells/mL or greater. When addition of microorganisms was discontinued (concentration of added microorganisms upon addition of 0), the concentration of oil content increased on the same day. Unlike normal microorganism culture, 100-fold or greater proliferation of added microorganisms was not found at any of the amounts added, despite oil and fat being decomposed. Furthermore, 10-fold or greater proliferation of added microorganisms was not found, even if oil content was effectively decomposed. At a concentration of added oil decomposing bacteria upon addition of 1 × 10⁶ cells/mL, a sufficient effect of oil decomposition and removal was attained. Significance in adding more microorganisms was hardly found.

The diluted wastewater initially contained about 4 × 10⁸ cells/mL of microorganisms (Δ symbol in Figure **12****)** besides the added microorganisms. There was no significant change in the concentration of all microorganisms after addition of an oil and fat decomposing microbial formulation. The concentration of added microorganisms in water flowing out was about 1% or less of the concentration of all microorganisms, such that the effect of decomposition was sufficiently exerted without the added microorganisms becoming the dominant or primary species.

### 7-4. Results in ultrahigh concentration oil and fat (n-Hex value of about 30000 mg/L) wastewater

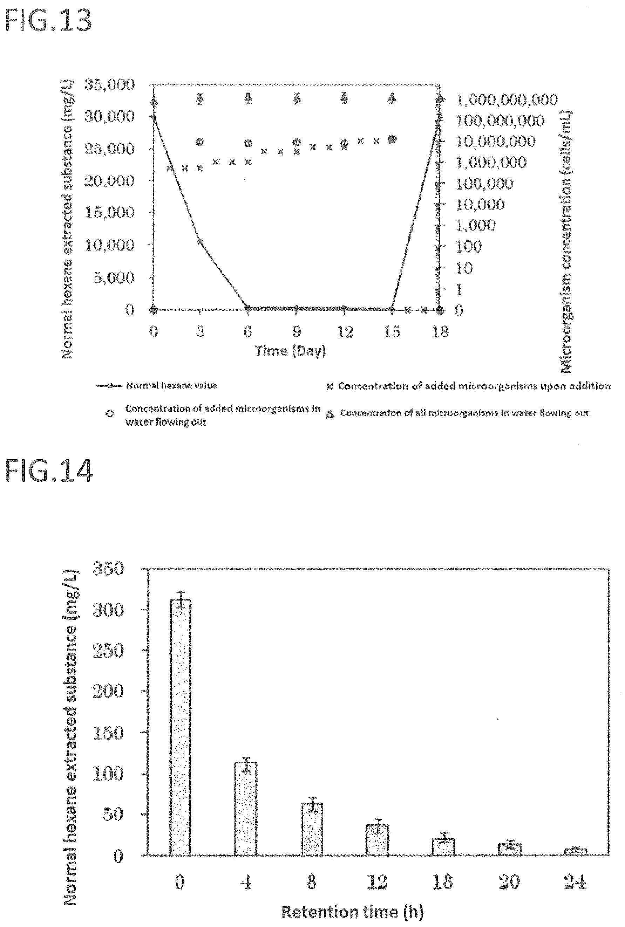

At a site with an n-Hex value level of 30000 mg/L and wastewater volume of 100 ton/day, tests were conducted at a 1/2000 scale at a treatment rate of 50 L/day. Water volume to be treated in the oil and fat decomposition vessel was set to 50 L, and the retention time was set to 24 hours. Microbial formulations with a concentration of 5 × 10⁸, 1 × 10⁹, 3 × 10⁹, 5 × 10⁹, or 1 × 10¹⁰ cells/mL were added continuously at a supply rate of 2.1 mL/h. The results are shown in Figure **13****.** The concentrations of added microorganisms upon addition were 5 × 10⁵, 1 × 10⁶, 3 × 10⁶, 5 × 10⁶, and 1 × 10⁷ cells/mL (× symbol).

As a result, in the same manner as intermittent addition, the n-Hex value decreased 99% or more at a concentration of added microorganisms upon addition of 1 × 10⁶ cells/mL or greater. When addition of microorganisms was discontinued (concentration of added microorganisms upon addition of 0), the concentration of oil content increased on the same day. Unlike normal microorganism culture, 100-fold or greater proliferation of added microorganisms was not found at any of the amounts added, despite oil and fat being decomposed. Furthermore, 10-fold or greater proliferation of added microorganisms was not found, even if oil content was effectively decomposed. The level of proliferation of microorganisms was similar even if the concentration of added microorganisms was changed. The proliferation was at most to the level of 1 × 10⁷ cells/mL. Thus, any more addition of microorganisms would be meaningless.

The wastewater initially contained about 1 × 10⁹ cells/mL of microorganisms (Δ symbol in Figure **13****)** besides the added microorganisms. There was no significant change in the concentration of all microorganisms after addition of an oil and fat decomposing microbial formulation. The concentration of added microorganisms in water flowing out was 1% or less of the concentration of all microorganisms, such that the effect of decomposition was sufficiently exerted without the added microorganisms becoming the dominant or primary species.

### Test Example 8: Effect of retention time

Decomposition tests that intermittently add a microbial formulation while allowing wastewater with low, moderate, high, and very high oil concentration (n-Hex value) to continuously flow were conducted. The same site verification demo version tester as that in Test Example 6 was installed at the site for the tests. The retention time was increased stepwise from 4 hours to 24 hours for 60 L of water volume to be treated of an oil and fat decomposition vessel, and a microbial formulation with a concentration corresponding to the oil and fat concentration was added at each retention time. Therefore, the treatment rate decreased stepwise from 360 L/day to 60 L/day. The same mixed formulation of Burkholderia arboris and Yarrowia lipolytica as that in Test Example 1 was used as a microbial formulation.

The water flowing out was sampled after the retention time had elapsed and immediately before the next microbial formulation addition, and the concentration of n-Hex extracted substance was measured in accordance with JIS K0102 to investigate the effect of decomposing and removing oil content. The pH in the oil and fat decomposition vessel was automatically adjusted to the vicinity of neutral. The water temperature was between 25 to 35°C. During the treatment, the samples were aerated and agitated so that DO would be 1 mg/L or greater. The n-Hex value was analyzed three times for each sample, and the standard error was found.

### 8-1. Results in low concentration oil and fat (n-Hex value of about 300 mg/L) wastewater

At a site with an n-Hex value level of 300 mg/L and wastewater volume of 100 ton/day, tests were conducted with actual wastewater continuously flowing to the demo version tester. 60 mL of microbial formulations with a concentration of 5 × 10⁷ cells/mL was added, which is 1/1000 of the water volume to be treated, at the start of each specified retention time. The results are shown in Figure **14****.** The concentration of added microorganisms upon addition was 5 × 10⁴ cells/mL. The n-Hex value decreased nearly 90% in a 12 hour retention time. Furthermore, the n-Hex value decreased 90% or more in 18 hour retention time to achieve less than 30 mg/L, which is the reference value of release into the sewage system in many municipalities, thus exhibiting a treatment effect that would render even the primary treatment, i.e., active sludge treatment, unnecessary in terms of just the n-Hex value.

### 8-2. Results in moderate concentration oil and fat (n-Hex value of about 3000 mg/L) wastewater

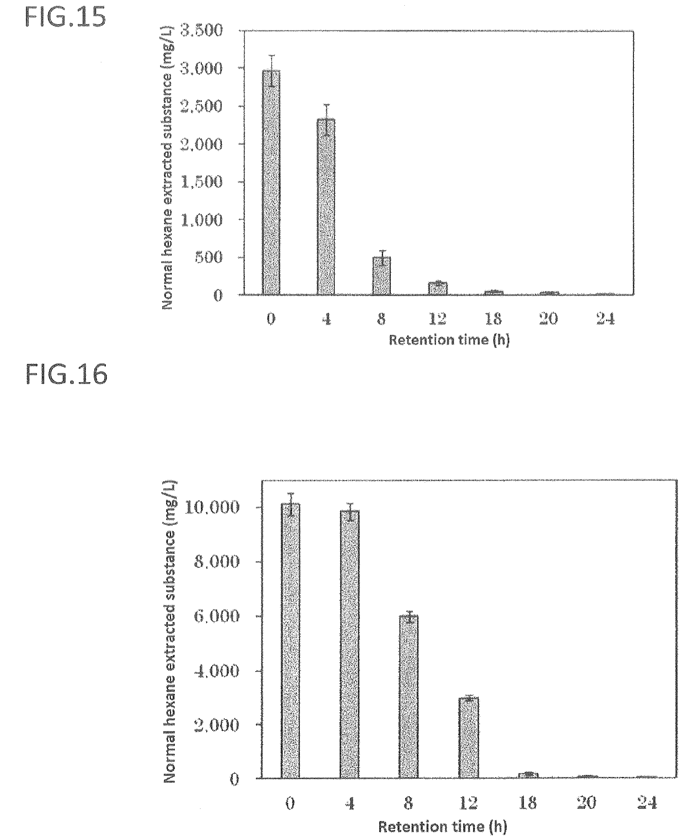

At a site with an n-Hex value level of 3000 mg/L and wastewater volume of 100 ton/day, tests were conducted with actual wastewater continuously flowing to the demo version tester. 60 mL of microbial formulations with a concentration of 5 × 10⁸ cells/mL was added, which is 1/1000 of the water volume to be treated, at the start of each specified retention time. The results are shown in Figure **15****.** The concentration of added microorganisms upon addition was 5 × 10⁵ cells/mL. The n-Hex value decreased 95% or more in an 18 hour retention time and 98% or more in 20 hour retention time to less than 50 mg/L, and 99% or more in 24 hours to less than to 30 mg/L less, which is the reference value of release into the sewage system in many municipalities.

### 8-3. Results in high concentration oil and fat (n-Hex value of about 10000 mg/L) wastewater

At a site with an n-Hex value level of 30000 mg/L and wastewater volume of 100 ton/day, tests were conducted with wastewater diluted 3-fold with groundwater continuously flowing to the demo version tester. 60 mL of microbial formulations with a concentration of 1 × 10⁹ cells/mL was added, which is 1/1000 of the water volume to be treated, at the start of each specified retention time. The results are shown in Figure **16****.** The concentration of added microorganisms upon addition was 1 × 10⁶ cells/mL. The n-Hex value decreased 99% or more in 20 hour retention time or more and decreased to less than 30 mg/L, which is the reference value of release into the sewage system in many municipalities, in 24 hours.

### 8-4. Results in ultrahigh concentration oil and fat (n-Hex value of about 30000 mg/L) wastewater

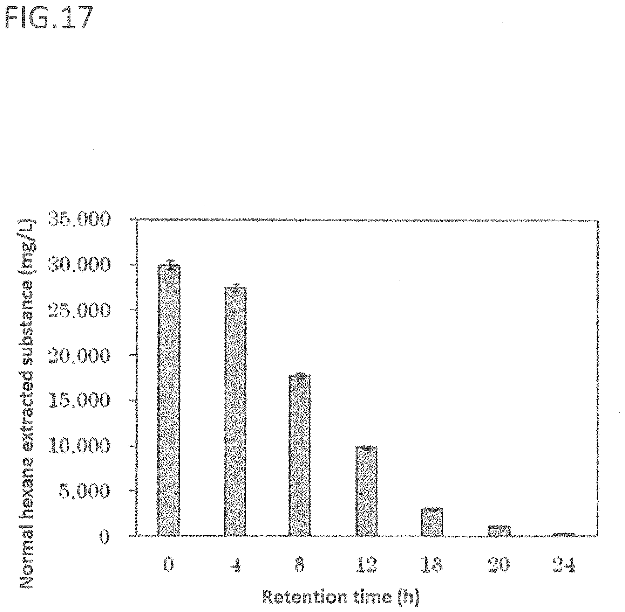

At a site with an n-Hex value level of 30000 mg/L and wastewater volume of 100 ton/day, tests were conducted with actual wastewater continuously flowing to the demo version tester. 60 mL of microbial formulations with a concentration of 3 × 10⁹ cells/mL was added, which is 1/1000 of the water volume to be treated, at the start of each specified retention time. The results are shown in Figure **17****.** The concentration of added microorganisms upon addition was 3 × 10⁶ cells/mL. The n-Hex value decreased 99% or more in a 24 hour retention time.

### Test Example 9: Relationship between amount of oil inflow and amount of added microorganism

In the same manner as Text Example 6, wastewater was treated with a plurality of amounts of oil to investigate the relationship between the amount of oil inflow and amount of added microorganism. The relationship between the amount of oil inflow and amount of added microorganism is shown below.

**[Table 1]**

| | | | | | | | |
|---|---|---|---|---|---|---|---|
| **Added microorganism/amount of oil * (cells/mg)** | **9,586** | **16,129** | **16,790** | **33,580** | **47,929** | **287,577** | **1,612,903** |
| **Residual oil ratio (%)** | **18** | **60** | **40** | **9** | **4** | **1** | **4** |

| | | | | | | | |
|---|---|---|---|---|---|---|---|
| *The amount of oil calculated from normal hexane value | | | | | | | |

The results in Table 1 revealed that sufficient decomposition of oil and fat is achieved when the amount of microbial formulation added to an oil and fat decomposition vessel is 3.0 × 10⁴ to 1.0 × 10⁸ cells, preferably about 3.0 × 10⁴ to 1 × 10⁷ cells per 1 mg of normal hexane extracted substance in oil- and fat-containing wastewater flowing into the oil and fat decomposition vessel.

## Claims

1. A method for the treatment of oil- and fat-containing wastewater, comprising decomposing oil and fat by continuously or intermittently adding a microbial formulation with an ability to decompose oil and fat to an oil and fat decomposition vessel where oil- and fat-containing wastewater continuously flows in and out, wherein an amount of the microbial formulation added is an amount that is effective for a microorganism to be established in the oil and fat decomposition vessel, and a microorganism other than the added microorganism comprised in the microbial formulation is present in oil- and fat-containing wastewater, comprising
measuring a normal hexane extracted substance in the oil- and fat-containing wastewater flowing into the oil and fat decomposition vessel, and determining the amount of the microbial formulation added in accordance with the normal hexane extracted substance, wherein the amount of the microbial formulation added is 3.0 × 10⁴ to 1.0 × 10⁷ cells per 1 mg of normal hexane extracted substance, wherein the amount of normal hexane extracted substance is the amount when determined in accordance with JIS K 0102,
wherein a dissolved oxygen concentration of oil- and fat-containing wastewater in the oil and fat decomposition vessel is 0.05 mg/L or greater, wherein a pH of oil- and fat-containing wastewater in the oil and fat decomposition vessel is within a range of 4.5 to 9.0, wherein a temperature of oil- and fat-containing wastewater in the oil and fat decomposition vessel is within a range of 12 to 42°C, wherein a hydraulic retention time (HRT) in the oil and fat decomposition vessel is 1 hour or greater,
wherein the wastewater comprises oil and fat at a concentration of 300 mg/L or greater of a normal hexane extracted substance on average, and
wherein the oil and fat decomposition vessel is free of a carrier.

2. The treatment method of claim 1, wherein the microbial formulation comprises at least one type of microorganism selected from the group consisting of a microorganism that produces lipase, and a microorganism that decomposes fatty acid and/or glycerol.

3. The treatment method of claim 1 or 2, wherein the microbial formulation comprises at least one species of microorganism selected from the group consisting of Bacillus bacteria, Corynebacterium bacteria, Rhodo-coccus bacteria, Burkholderia bacteria, Acinetobacter bacteria, Pseudomonas bacteria, Alcaligenes bacteria, Rhodobacter bacteria, Ralstonia bacteria, Acidovorax bacteria, Serratia bacteria, Flavobacterium bacteria, Candida yeast, Yarrowia yeast, Cryptococcus yeast, Tri-chosporon yeast, and Hansenula yeast.

4. The treatment method of any one of claims 1 to 3, wherein the microbial formulation is intermittently added, wherein a hydraulic retention time (HRT) in the oil and fat decomposition vessel is 12 hours or greater, and wherein the interval for adding the microbial formulation is between the HRT and 24 hours.

5. The treatment method of any one of claims 1 to 4, wherein an HRT in the oil and fat decomposition vessel is 12 hours or greater and 48 hours or less.

6. The treatment method of any one of claims 1 to 5, wherein C/N of oil- and fat-containing wastewater in the oil and fat decomposition vessel is within a range of 2 to 50.

7. The treatment method of any one of claims 1 to 6, wherein N/P of oil- and fat-containing wastewater in the oil and fat decomposition vessel is within a range of 1 to 20.

8. The treatment method of any one of claims 1-7, wherein the concentration of added microorganism in water flowing out from the oil and fat decomposition vessel is 0.1-fold to 10-fold of the concentration of added microorganism upon addition to the oil and fat decomposition vessel.

9. The treatment method of any one of claims 1-8, wherein an amount of the added microorganism in water flowing out from the oil and fat decomposition vessel is 1 × 10⁸ cells/mL or less.

## Patentansprüche

1. Verfahren zur Behandlung von öl- und fetthaltigem Abwasser, bei dem Öl und Fett durch kontinuierliche oder intermittierende Zugabe einer mikrobiellen Formulierung abgebaut werden, die die Fähigkeit hat, Öl und Fett abzubauen, zu einem Öl- und Fett Zersetzungsbehälter, in das öl- und fetthaltiges Abwasser kontinuierlich ein- und davon abfließt, wobei eine Menge der zugegebenen mikrobiellen Formulierung eine Menge ist, die zur Etablierung eines Mikroorganismus in dem Öl- und Fett-Zersetzungsbehälter wirksam ist, und wobei in dem in öl- und fetthaltigem Abwasser ein anderer Mikroorganismus als der zugegebene Mikroorganismus, der in der mikrobiellen Formulierung enthalten ist, vorhanden ist, wobei weiterhin
eine mit normal-Hexan extrahierte Substanz in dem öl- und fetthaltigen Abwasser, das in das Öl- und Fett-Zersetzungsbehälter fließt, gemessen wird und die Menge der zugesetzten mikrobiellen Formulierung in Übereinstimmung mit der mit normal-Hexan extrahierten Substanz bestimmt wird, wobei die Menge der zugesetzten mikrobiellen Formulierung 3,0 × 10⁴ bis 1,0 × 10⁷ Zellen pro 1 mg der mit normal-Hexan extrahierten Substanz ist, wobei die Menge der mit normal-Hexan extrahierten Substanz eine Menge ist, die in Übereinstimmung mit JIS K 0102 bestimmt wird,
wobei die Konzentration an gelöstem Sauerstoff des öl- und fetthaltigen Abwassers in dem Öl- und Fett-Zersetzungsbehälter 0,05 mg/L oder mehr beträgt, wobei der pH-Wert des öl- und fetthaltigen Abwassers in dem Öl- und Fett-Zersetzungsbehälter in einem Bereich von 4,5 bis 9,0 liegt, wobei die Temperatur des öl- und fetthaltigen Abwassers in dem Öl- und Fett-Zersetzungsbehälter in einem Bereich von 12 bis 42°C liegt, wobei eine hydraulische Verweilzeit (HRT) in dem Öl- und Fett-Zersetzungsbehälter 1 Stunde oder mehr beträgt,
wobei das Abwasser Öl und Fett in einer durchschnittlichen Konzentration von 300 mg/L oder mehr einer mit normal- Hexan extrahierten Substanz enthält, und
wobei der Öl- und Fett-Zersetzungsbehälter frei von einem Trägerstoff ist.

2. Verfahren zur Behandlung nach Anspruch 1, wobei die mikrobielle Formulierung mindestens eine Art von Mikroorganismus umfasst, der aus der Gruppe ausgewählt ist, die aus einem Mikroorganismus besteht, der Lipase produziert, und einem Mikroorganismus, der Fettsäure und/oder Glycerin abbaut.

3. Verfahren zur Behandlung nach Anspruch 1 oder 2, wobei die mikrobielle Formulierung mindestens eine Spezies von Mikroorganismen umfasst, die aus der Gruppe ausgewählt ist, die aus Bacillus-Bakterien, Corynebacterium-Bakterien, Rhodococcus-Bakterien, Burkholderia-Bakterien, Acinetobacter-Bakterien, Pseudomonas-Bakterien, Alcaligenes-Bakterien, Rhodobacter-Bakterien, Ralstonia-Bakterien, Acidovorax-Bakterien, Serratia-Bakterien, Flavobacterium-Bakterien, Candida-Hefe, Yarrowia-Hefe, Cryptococcus-Hefe, Trichosporon-Hefe und Hansenula-Hefe besteht.

4. Verfahren zur Behandlung nach einem der Ansprüche 1 bis 3, wobei die mikrobielle Formulierung intermittierend zugegeben wird, wobei eine hydraulische Verweilzeit (HRT) in dem Öl- und Fett-Zersetzungsbehälter 12 Stunden oder mehr beträgt und wobei das Intervall für die Zugabe der mikrobiellen Formulierung zwischen der HRT und 24 Stunden liegt.

5. Verfahren zur Behandlung nach einem der Ansprüche 1 bis 4, wobei eine HRT im Öl- und Fett-Zersetzungsbehälter 12 Stunden oder mehr und 48 Stunden oder weniger beträgt.

6. Verfahren zur Behandlung nach einem der Ansprüche 1 bis 5, wobei der C/N-Wert des öl- und fetthaltigen Abwassers im Öl- und Fett-Zersetzungsbehälter im Bereich von 2 bis 50 liegt.

7. Verfahren zur Behandlung nach einem der Ansprüche 1 bis 6, wobei das N/P des öl- und fetthaltigen Abwassers im Öl- und Fett-Zersetzungsbehälter im Bereich von 1 bis 20 liegt.

8. Verfahren zur Behandlung nach einem der Ansprüche 1 bis 7, wobei die Konzentration der zugesetzten Mikroorganismen im Wasser, das aus dem Öl- und Fett-Zersetzungsbehälter abfließt, das 0,1- bis 10-fache der Konzentration der zugesetzten Mikroorganismen bei der Zugabe in das Öl- und Fett-Zersetzungsbehälter beträgt.

9. Verfahren zur Behandlung nach einem der Ansprüche 1 bis 8, wobei die Menge des zugesetzten Mikroorganismus im Wasser, das aus dem Öl- und Fett-Zersetzungsbehälter abfließt, 1 × 10⁸ Zellen/mL oder weniger beträgt.

## Revendications

1. Procédé de traitement d'eaux usées contenant de l'huile et de la graisse, comprenant la décomposition de l'huile et de la graisse par l'ajout continu ou intermittent d'une formulation microbienne ayant une capacité de décomposer de l'huile et de la graisse dans un récipient de décomposition d'huile et de graisse où des eaux usées contenant de l'huile et de la graisse s'écoulent en continu à l'intérieur et hors de celui-ci, dans lequel une quantité de la formulation microbienne ajoutée est une quantité qui est efficace pour qu'un micro-organisme soit installé dans le récipient de décomposition d'huile et de graisse, et pour qu'un micro-organisme autre que le micro-organisme ajouté compris dans la formulation microbienne soit présent dans des eaux usées contenant de l'huile et de la graisse, comprenant
la mesure d'une substance extraite par hexane normal dans les eaux usées contenant de l'huile et de la graisse s'écoulant dans le récipient de décomposition d'huile et de graisse, et la détermination de la quantité de la formulation microbienne ajoutée conformément à la substance extraite par hexane normal, dans lequel la quantité de la formulation microbienne ajoutée est de 3,0 × 10⁴ à 1,0 × 10⁷ cellules pour 1 mg de substance extraite par hexane normal, dans lequel la quantité de substance extraite par hexane normal est la quantité lorsque déterminée conformément à la norme JIS K 0102,
dans lequel une concentration en oxygène dissous d'eaux usées contenant de l'huile et de la graisse dans le récipient de décomposition d'huile et de graisse est de 0,05 mg/l ou plus, dans lequel un pH d'eaux usées contenant de l'huile et de la graisse dans le récipient de décomposition d'huile et de graisse est dans une plage de 4,5 à 9,0, dans lequel une température d'eaux usées contenant de l'huile et de la graisse dans le récipient de décomposition d'huile et de graisse est dans une plage de 12 à 42 °C, dans lequel un temps de rétention hydraulique (HRT) dans le récipient de décomposition d'huile et de graisse est de 1 heure ou plus,
dans lequel les eaux usées comprennent de l'huile et de la graisse à une concentration de 300 mg/l ou plus d'une substance extraite par hexane normal en moyenne, et
dans lequel le récipient de décomposition d'huile et de graisse est exempt d'un support.

2. Procédé de traitement selon la revendication 1, dans lequel la formulation microbienne comprend au moins un type de micro-organisme choisi dans le groupe consistant en un micro-organisme qui produit une lipase, et un micro-organisme qui décompose un acide gras et/ou du glycérol.

3. Procédé de traitement selon la revendication 1 ou 2, dans lequel la formulation microbienne comprend au moins une espèce de micro-organisme choisie dans le groupe consistant en bactérie Bacillus, bactérie Corynebacterium, bactérie Rhodo-coccus, bactérie Burkholderia, bactérie Acinetobacter, bactérie Pseudomonas, bactérie Alcaligenes, bactérie Rhodobacter, bactérie Ralstonia, bactérie Acidovorax, bactérie Serratia, bactérie Flavobacterium, levure Candida, levure Yarrowia, levure Cryptococcus, levure Tri-chosporon, et levure Hansenula.

4. Procédé de traitement selon l'une quelconque des revendications 1 à 3, dans lequel la formulation microbienne est ajoutée par intermittence, dans lequel un temps de rétention hydraulique (HRT) dans le récipient de décomposition d'huile et de graisse est de 12 heures ou plus, et dans lequel l'intervalle d'ajout de la formulation microbienne est compris entre le HRT et 24 heures.

5. Procédé de traitement selon l'une quelconque des revendications 1 à 4, dans lequel un HRT dans le récipient de décomposition d'huile et de graisse est de 12 heures ou plus et de 48 heures ou moins.

6. Procédé de traitement selon l'une quelconque des revendications 1 à 5, dans lequel le rapport C/N d'eaux usées contenant de l'huile et de la graisse dans le récipient de décomposition d'huile et de graisse est dans une plage de 2 à 50.

7. Procédé de traitement selon l'une quelconque des revendications 1 à 6, dans lequel le rapport N/P d'eaux usées contenant de l'huile et de la graisse dans le récipient de décomposition d'huile et de graisse est dans une plage de 1 à 20.

8. Procédé de traitement selon l'une quelconque des revendications 1 à 7, dans lequel la concentration de micro-organisme ajouté dans de l'eau s'écoulant hors du récipient de décomposition d'huile et de graisse est de 0,1 fois à 10 fois la concentration de micro-organisme ajouté lors de l'ajout au récipient de décomposition d'huile et de graisse.

9. Procédé de traitement selon l'une quelconque des revendications 1 à 8, dans lequel une quantité du micro-organisme ajouté dans de l'eau s'écoulant hors du récipient de décomposition d'huile et de graisse est de 1 × 10⁸ cellules/ml ou moins.
